(19) Europäisches Patentamt
European Patent Office
Office européen des brevets

(11) EP 1 131 302 B1

(12) EUROPÄISCHE PATENTSCHRIFT

(45) Veröffentlichungstag und Bekanntmachung des Hinweises auf die Patenterteilung:
**01.02.2006 Patentblatt 2006/05**

(21) Anmeldenummer: **99972626.8**

(22) Anmeldetag: **03.11.1999**

(51) Int Cl.:
***C07D 239/70*** *(2006.01)* ***C07D 401/12*** *(2006.01)*
***A61K 31/517*** *(2006.01)*

(86) Internationale Anmeldenummer:
**PCT/EP1999/008382**

(87) Internationale Veröffentlichungsnummer:
**WO 2000/031047 (02.06.2000 Gazette 2000/22)**

# (54) SUBSTITUIERTE 4-AMINO-2-ARYL-CYCLOPENTA [D]PYRIMIDINE, IHRE HERSTELLUNG, IHRE VERWENDUNG UND SIE ENTHALTENDE PHARMAZEUTISCHE PRÄPARATE

SUBSTITUTED 4-AMINO-2-ARYL-CYCLOPENTA [D]PYRIMIDINES, THEIR PRODUCTION AND USE AND PHARMACEUTICAL PREPARATIONS CONTAINING THE SAME

4-AMINO-2-ARYL-CYCLOPENTA [D]PYRIMIDINE SUBSTITUEE, SA FABRICATION, SON UTILISATION ET PREPARATIONS PHARMACEUTIQUES LA RENFERMANT

(84) Benannte Vertragsstaaten:
**AT BE CH CY DE DK ES FI FR GB GR IE IT LI LU MC NL PT SE**

(30) Priorität: **19.11.1998 DE 19853278**

(43) Veröffentlichungstag der Anmeldung:
**12.09.2001 Patentblatt 2001/37**

(73) Patentinhaber: **Sanofi-Aventis Deutschland GmbH**
**65929 Frankfurt am Main (DE)**

(72) Erfinder:
- **SCHINDLER, Ursula**
  **D-65812 Bad Soden (DE)**
- **SCHÖNAFINGER, Karl**
  **D-63755 Alzenau (DE)**
- **STROBEL, Hartmut**
  **D-65835 Liederbach (DE)**

(56) Entgegenhaltungen:
**DE-A- 4 029 654**

- **PATENT ABSTRACTS OF JAPAN vol. 1995, no. 11, 26. Dezember 1995 (1995-12-26) & JP 07 228573 A (DAINIPPON PHARMACEUT CO LTD), 29. August 1995 (1995-08-29) in der Anmeldung erwähnt**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Die vorliegende Erfindung betrifft Verbindungen der Formel I,

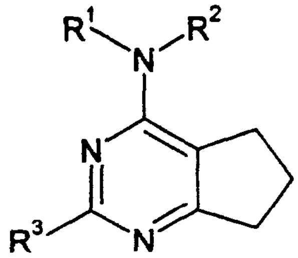

I

in der $R^1$, $R^2$ und $R^3$ die unten angegebenen Bedeutungen haben, die wertvolle Arzneimittelwirkstoffe für die Therapie und Prophylaxe von Krankheiten sind, zum Beispiel von Herz-Kreislauferkrankungen wie Bluthochdruck, Angina pectoris, Herzinsuffizienz, Thrombosen oder Atherosklerose. Die Verbindungen der Formel I haben die Fähigkeit zur Modulation der körpereigenen Produktion von cyclischem Guanosinmonophosphat (cGMP) und eignen sich generell zur Therapie und Prophylaxe von Krankheitszuständen, die mit einem gestörten cGMP-Haushalt verbunden sind. Die Erfindung betrifft weiterhin Verfahren zur Herstellung von Verbindungen der Formel I, ihre Verwendung zur Therapie und Prophylaxe der bezeichneten Krankheitszustände und zur Herstellung von Arzneimitteln dafür, sowie pharmazeutische Präparate, die Verbindungen der Formel I enthalten.

cGMP ist ein wichtiger intrazellulärer Botenstoff, der über die Modulation cGMPabhängiger Proteinkinasen, Phosphodiesterasen und Ionenkanäle eine Reihe von pharmakologischen Effekten auslöst. Beispiele sind die Glattmuskelrelaxation, die Inhibition der Thrombozytenaktivierung und die Hemmung von Glattmuskelzellproliferation und Leukozytenadhäsion. cGMP wird durch partikuläre und lösliche Guanylatcyclasen als Antwort auf eine Reihe extrazellulärer und intrazellulärer Stimuli produziert. Im Falle der partikulären Guanylatcyclasen erfolgt die Stimulation im wesentlichen durch peptidische Signalstoffe, wie dem atrialen natriuretischen Peptid oder dem cerebralen natriuretischen Peptid. Die löslichen Guanylatcyclasen (sGC), bei denen es sich um cytosolische, heterodimere Hämproteine handelt, werden dagegen im wesentlichen durch eine Familie niedermolekularer, enzymatisch gebildeter Faktoren reguliert. Wichtigstes Stimulans ist das Stickstoffmonoxid (NO) oder eine nahe verwandte Spezies. Die Bedeutung anderer Faktoren wie Kohlenmonoxid oder dem Hydroxylradikal ist noch weitgehend ungeklärt. Als Aktivierungsmechanismus der Aktivierung durch NO wird die Anbindung von NO an das Häm unter Ausbildung eines pentakoordinierten Häm-Nitrosyl-Komplexes diskutiert. Die damit verbundene Freisetzung des im Basal-Zustand an das Eisen gebundenen Histidins überführt das Enzym in die aktivierte Konformation.

Aktive lösliche Guanylatcyclasen setzen sich aus je einer $\alpha$- und einer $\beta$-Untereinheit zusammen. Von den Untereinheiten wurden mehrere Subtypen beschrieben, die sich untereinander bezüglich Sequenz, gewebespezifischer Verteilung und Expression in verschiedenen Entwicklungsstadien unterscheiden. Die Subtypen $\alpha_1$ und $\beta_1$ werden hauptsächlich in Gehirn und Lunge exprimiert, während $\beta_2$ vor allem in Leber und Niere gefunden wird. In humanem fötalen Gehirn konnte der Subtyp $\alpha_2$ nachgewiesen werden. Die als $\alpha_3$ und $\beta_3$ bezeichneten Untereinheiten wurden aus menschlichem Gehim isoliert und sind homolog zu $\alpha_1$ und $\beta_1$. Neuere Arbeiten weisen auf eine $\alpha_{2i}$-Untereinheit hin, die ein Insert in der katalytischen Domäne enthält. Alle Untereinheiten zeigen große Homologien im Bereich der katalytischen Domäne. Die Enzyme enthalten vermutlich ein Häm pro Heterodimer, das über $\beta_1$-Cys-78 und/oder $\beta_1$-His-105 gebunden ist und Teil des regulatorischen Zentrums ist.

Unter pathologischen Bedingungen kann die Bildung Guanylatcyclase-aktivierender Faktoren vermindert sein oder es kann durch das vermehrte Auftreten freier Radikale ein verstärkter Abbau derselben erfolgen. Die daraus resultierende verminderte Aktivierung der sGC führt über die Abschwächung der jeweiligen cGMPvermittelten Zellantwort beispielsweise zum Anstieg des Blutdrucks, zur Plättchenaktivierung oder zu vermehrter Zellproliferation und Zelladhäsion. Als Folge kommt es zur Ausbildung von endothelialer Dysfunktion, Atherosklerose, Bluthochdruck, stabiler und instabiler Angina pectoris, Thrombosen, Myocardinfarkt, Schlaganfällen oder erektiler Dysfunktion. Die pharmakologische Stimulation der sGC bietet eine Möglichkeit zur Normalisierung der cGMP-Produktion und erlaubt damit die Behandlung bzw. Prävention derartiger Krankheiten.

Zur pharmakologischen Stimulation der sGC wurden bisher fast ausschließlich Verbindungen verwendet, deren Wirkung auf einer intermediären NO-Freisetzung beruht, beispielsweise organische Nitrate. Der Nachteil dieser Behandlungsweise liegt in der Toleranzentwicklung und Wirkungsabschwächung und der deshalb erforderlich werdenden höheren Dosierung.

Verschiedene nicht über eine NO-Freisetzung wirkende sGC-Stimulatoren wurden von Vesely in einer größeren Zahl von Arbeiten beschrieben. Die Verbindungen, bei denen es sich zumeist um Hormone, Pflanzenhormone, Vitamine oder zum Beispiel Naturstoffe wie Echsengifte handelt, zeigen jedoch durchweg nur schwache Effekte auf die cGMP-Bildung in Zellysaten (D. L. Vesely, Eur. J. Clin. Invest. 15 (1985) 258; D. L. Vesely, Biochem. Biophys. Res. Comm. 88 (1979) 1244). Eine Stimulation Häm-freier Guanylatcyclase durch Protoporphyrin IX wurde durch Ignarro et al. (Adv. Pharmacol. 26 (1994) 35) nachgewiesen. Pettibone et al. (Eur. J. Pharmacol. 116 (1985) 307) beschrieben für Diphenyliodoniumhexafluorophoshat eine blutdrucksenkende Wirkung und führten diese auf eine Stimulation der sGC zurück. Isoliquiritiginin, das an isolierten Rattenaorten eine relaxierende Wirkung zeigt, aktiviert laut Yu et al. (Brit. J. Pharmacol. 114 (1995) 1587) ebenfalls die sGC. Ko et al. (Blood 84 (1994) 4226), Yu et al. (Biochem. J. 306 (1995) 787) und Wu et al. (Brit. J. Pharmacol. 116 (1995) 1973) wiesen eine sGC-stimulierende Aktivität von 1-Benzyl-3-(5-hydroxymethyl-2-furyl)-indazol nach und demonstrierten eine antiproliferative und thrombozytenhemmende Wirkung. Für verschiedene Indazole wird in der EP-A-667 345 eine inhibierende Wirkung auf die Plättchenaggregation beschrieben; heterocyclylmethylsubstituierte und benzylsubstituierte Pyrazole werden weiterhin in der WO-A-98/16 507 und der WO-A-98/16 223 beschrieben. In der internationalen Patentanmeldung PCT/EP99/05636 werden Pyrimidine beschrieben, die eine sGC-stimulierende Aktivität zeigen.

Bestimmte Cycloalkano[d]pyrimidine und Cyclopenta[d]pyrimidine sind bereits bekannt. So werden in der DE-A-40 29 654 fungizid wirkende 2-Phenyl-cycloalkanopyrimidine beschrieben, die in der 4-Position spezielle Aminosubstituenten tragen, die Alkinylgruppen enthalten. In der US-A-3 346 452 und der US-A-3 322 759 werden Cycloalkanopyrimidine beschrieben, die in der 4-Position eine Aminoalkylaminogruppe tragen und die analgetische Wirkungen aufweisen. In der WO-A-97/47 601 werden spezielle bicyclische Pyrimidine beschrieben, die als Dopamin-Rezeptor-Antagonisten wirken und beispielsweise zur Behandlung der Schizophrenie eingesetzt werden können und die einen Heterocyclylalkylamino-Substituenten tragen, in dem der Heterocyclus über ein Ringstickstoffatom gebunden ist. In der JP-A-07/228 573 werden 2-Phenyl-cycloalkanopyrimidine beschrieben, die Serotonin-Rezeptor-Antagonisten sind und sich als Psychopharmaka eignen und die in der 4-Position einen Piperazinosubstituenten oder Homopiperazinosubstituenten tragen. In der EP-A-826 673 werden 2-Phenyl-cycloalkanopyrimidine beschrieben, die auf Benzodiazepin-Rezeptoren wirken und zum Beispiel eine anxiolytische Wirkung haben und die in der 4-Position spezielle Aminosubstituenten tragen, die Aminocarbonylgruppen enthalten.

Überraschend wurde nun gefunden, daß die erfindungsgemäßen Pyrimidine der Formel I eine starke Guanylatcyclase-Aktivierung bewirken, aufgrund derer sie zur Therapie und Prophylaxe von Krankheiten geeignet sind, die mit einem niedrigen cGMP-Spiegel verbunden sind.

Die vorliegende Erfindung betrifft somit Verbindungen der Formel I,

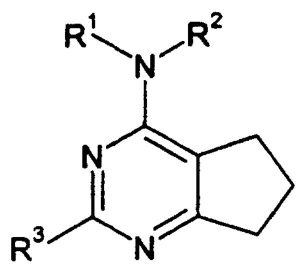

I

in der

$R^1$ und $R^2$, die unabhängig voneinander sind und gleich oder verschieden sein können, für Wasserstoff stehen, oder für $(C_1-C_8)$-Alkyl stehen, das durch einen oder mehrere gleiche oder verschiedene Substituenten aus der Reihe Hydroxy, $(C_1-C_4)$-Alkoxy, $(C_1-C_4)$-Alkyl-$S(O)_m$-, Phenyl, Naphthyl und Pyridyl substituiert sein kann, oder für $(C_3-C_9)$-Cycloalkyl stehen, das durch einen oder mehrere gleiche oder verschiedene Substituenten aus der Reihe $(C_1-C_4)$-Alkyl, Hydroxy, Amino und Benzyl substituiert sein kann, oder für den Rest eines 5-gliedrigen bis 7-gliedrigen gesättigten heterocyclischen Ringes stehen, der ein oder zwei gleiche oder verschiedene Hetero-Ringglieder aus der Reihe O, $NR^{10}$ und $S(O)_m$ enthält und der durch einen oder mehrere gleiche oder verschiedene Substituenten aus der Reihe $(C_1-C_4)$-Alkyl, Hydroxy und Aryl-$(C_1-C_4)$-alkyl- substituiert sein kann, wobei in den Resten $R^1$ oder $R^2$ enthaltene Reste Phenyl, Naphthyl, Pyridyl und Benzyl unsubstituiert sein können oder im aromatischen Ring durch einen oder mehrere gleiche oder verschiedene Substituenten aus der Reihe Halogen, $(C_1-C_4)$-Alkyl, Phenyl, $CF_3$, $NO_2$, OH, -O-$(C_1-C_4)$-Alkyl, -O-$(C_2-C_4)$-Alkyl-O-$(C_1-C_4)$-alkyl, $(C_1-C_2)$-Alkylendioxy, $NH_2$, -NH-$(C_1-C_4)$-Alkyl,

-N(($C_1$-$C_4$)-Alkyl)$_2$, -NH-CHO, -NH-CO-($C_1$-$C_4$)-Alkyl, -CN, -CO-$NH_2$, -CO-NH-($C_1$-$C_4$)-Alkyl, -CO-N(($C_1$-$C_4$)-Alkyl)$_2$, -CO-OH, -CO-O-($C_1$-$C_4$)-Alkyl, -CHO und -CO-($C_1$-$C_4$)-Alkyl substituiert sein können, wobei aber $R^1$ und $R^2$ nicht beide gleichzeitig für Wasserstoff stehen können;

oder

der Rest $R^1R^2N$- für einen über ein Ringstickstoffatom gebundenen Rest eines 5-gliedrigen bis 7-gliedrigen gesättigten heterocyclischen Ringes steht, der neben dem die Reste $R^1$ und $R^2$ tragenden Stickstoffatom ein weiteres Hetero-Ringglied aus der Reihe O und $S(O)_m$ enthalten kann und der durch einen oder mehrere gleiche oder verschiedene Substituenten aus der Reihe ($C_1$-$C_4$)-Alkyl, Hydroxy, ($C_1$-$C_4$)-Alkoxy und $R^{11}R^{12}N$ substituiert sein kann;

$R^3$ für Aryl steht, aber nicht für unsubstituiertes Phenyl stehen kann;

$R^{10}$ für Wasserstoff, ($C_1$-$C_4$)-Alkyl, Aryl-($C_1$-$C_4$)-alkyl-, Hydroxy-($C_1$-$C_4$)-alkyl-, Hydroxycarbonyl-($C_1$-$C_4$)-alkyl-, (($C_1$-$C_4$)-Alkoxycarbonyl)-($C_1$-$C_4$-alkyl-, $R^{11}R^{12}N$-CO-($C_1$-$C_4$)-alkyl-, $R^{13}$-$SO_2$- oder Aryl steht;

$R^{11}$ und $R^{12}$ für gleiche oder verschiedene Reste aus der Reihe Wasserstoff und ($C_1$-$C_4$)-Alkyl stehen;

$R^{13}$ für ($C_1$-$C_4$)-Alkyl, Aryl oder $R^{11}R^{12}N$ steht;

Aryl für Phenyl, Naphthyl oder Heteroaryl steht, die alle durch einen oder mehrere gleiche oder verschiedene Substituenten aus der Reihe Halogen, ($C_1$-$C_4$)-Alkyl, Phenyl, $CF_3$, $NO_2$, OH, -O-($C_1$-$C_4$)-Alkyl, -O-($C_2$-$C_4$)-Alkyl-O-($C_1$-$C_4$)-alkyl, ($C_1$-$C_2$)-Alkylendioxy, $NH_2$, -NH-($C_1$-$C_4$)-Alkyl, -N(($C_1$-$C_4$)-Alkyl)$_2$, -NH-CHO, -NH-CO-($C_1$-$C_4$)-Alkyl, -CN, -CO-$NH_2$, -CO-NH-($C_1$-$C_4$)-Alkyl, -CO-N(($C_1$-$C_4$)-Alkyl)$_2$; -CO-OH, -CO-O-($C_1$-$C_4$)-Alkyl, -CHO und -CO-($C_1$-$C_4$)-Alkyl substituiert sein können;

Heteroaryl für den Rest eines monocyclischen 5-gliedrigen oder 6-gliedrigen aromatischen Heterocyclus oder eines bicyclischen 8-gliedrigen bis 10-gliedrigen aromatischen Heterocyclus steht, die jeweils ein oder mehrere gleiche oder verschiedene Ringheteroatome aus der Reihe N, O und S enthalten;

m für 0, 1 oder 2 steht;

wobei geeignete Stickstoffatome in den Verbindungen der Formel I auch als N-Oxide vorliegen können;

in allen ihren stereoisomeren Formen und Mischungen davon in allen Verhältnissen, und ihre physiologisch verträglichen Salze.

Können Gruppen oder Substituenten mehrfach in den Verbindungen der Formel 1 vorkommen, so können sie alle unabhängig voneinander die angegebenen Bedeutungen haben und können jeweils gleich oder verschieden sein.

Alkylreste können geradkettig oder verzweigt sein. Dies gilt auch, wenn sie in anderen Gruppen enthalten sind, zum Beispiel in Alkoxygruppen, Alkoxycarbonylgruppen oder Aminogruppen, oder wenn sie substituiert sind. Beispiele für Alkylgruppen sind Methyl, Ethyl, Propyl, Butyl, Pentyl, Hexyl, Heptyl, Octyl, die n-Isomeren dieser Reste, Isopropyl, Isobutyl, Isopentyl, sec-Butyl, tert-Butyl, Neopentyl, 3,3-Dimethylbutyl. Sind Alkylreste durch einen oder mehrere Substituenten substituiert, so sind sie bevorzugt durch einen, zwei oder drei, insbesondere durch einen oder zwei, gleiche oder verschiedene Substituenten substituiert. Substituenten können sich an beliebigen Kohlenstoffatomen des Alkylrestes befinden.

Cycloalkyl ist beispielsweise Cyclopropyl, Cyclobutyl, Cyclopentyl, Cyclohexyl, Cycloheptyl, Cyclooctyl oder Cyclononyl, die alle auch wie angegeben substituiert sein können, also zum Beispiel durch einen oder mehrere gleiche oder verschiedene ($C_1$-$C_4$)-Alkylreste, insbesondere durch Methyl, und/oder durch Hydroxy. Sind Cycloalkylreste durch einen oder mehrere Substituenten substituiert, so sind sie bevorzugt durch einen, zwei, drei oder vier, insbesondere durch einen oder zwei, gleiche oder verschiedene Substituenten substituiert. Beispiele für solche substituierten Cycloalkylreste sind 4-Methylcyclohexyl, 4-tert-Butylcyclohexyl, 4-Hydroxycyclohexyl, 4-Aminocyclohexyl oder 2,3-Dimethylcyclopentyl. Substituenten können sich an beliebigen Kohlenstoffatomen des Cycloalkylrestes befinden.

Carbocyclische Arylreste wie Phenylreste und Naphthylreste und Heteroarylreste, können, soweit nicht anders angegeben, unsubstituiert sein oder einen oder mehrere, zum Beispiel ein, zwei, drei oder vier, gleiche oder verschiedene Substituenten tragen, die sich in beliebigen Positionen befinden können. Soweit nicht anders angegeben, können in diesen Resten als Substituenten zum Beispiel die in der Definition der Gruppe Aryl angegebenen Substituenten auftreten. Sind in Verbindungen der Formel I Nitrogruppen als Substituenten vorhanden, so sind bevorzugt insgesamt nur bis zu zwei Nitrogruppen im Molekül vorhanden. Trägt ein Arylrest wie zum Beispiel ein Phenylrest wiederum einen Phenylrest als Substituenten, so kann in letzterem der Benzolring auch wiederum unsubstituiert sein oder durch einen oder mehrere, zum Beispiel ein, zwei, drei oder vier, gleiche oder verschiedene Reste substituiert sein, zum Beispiel durch Reste aus der Reihe ($C_1$-$C_4$)-Alkyl, Halogen, Hydroxy, ($C_1$-$C_4$)-Alkoxy, Trifluormethyl, Cyan, Hydroxycarbonyl, (($C_1$-$C_4$)-Alkoxy)carbonyl, Aminocarbonyl, Nitro, Amino, ($C_1$-$C_4$)-Alkylamino, Di-(($C_1$-$C_4$)-alkyl)amino und (($C_1$-$C_4$)-Alkyl)carbonylamino.

In monosubstituierten Phenylresten kann sich der Substituent in der 2-Position, der 3-Position oder der 4-Position befinden, in disubstituierten Phenylresten können sich die Substituenten in 2,3-Position, 2,4-Position, 2,5-Position, 2,6-Position, 3,4-Position oder 3,5-Position befinden. In trisubstituierten Phenylresten können sich die Substituenten in 2,3,4-Position, 2,3,5-Position, 2,3,6-Position, 2,4,5-Position, 2,4,6-Position oder 3,4,5-Position befinden. Naphthyl kann 1-Naphthyl oder 2-Naphthyl sein. In monosubstituierten 1-Naphthylresten kann sich der Substituent in der 2-Position,

der 3-Position, der 4-Position, der 5-Position, der 6-Position, der 7-Position oder der 8-Position befinden, in monosubstituierten 2-Naphthylresten in der 1-Position, der 3-Position, der 4-Position, der 5-Position, der 6-Position, der 7-Position oder der 8-Position. Auch in mehrfach substituierten Naphthylresten, zum Beispiel zweifach oder dreifach substituierten Naphthylresten, können sich die Substituenten in allen möglichen Positionen befinden.

Soweit nicht anders angegeben, leiten sich Heteroarylreste, Reste von gesättigten heterocyclischen Ringen und Reste von Ringen; die von zwei an ein Stickstoffatom gebundenen Gruppen zusammen mit diesem Stickstoffatom gebildet werden, bevorzugt von Heterocyclen ab, die ein, zwei, drei oder vier gleiche oder verschiedene Ringheteroatome enthalten, besonders bevorzugt von Heterocyclen, die ein oder zwei oder drei, insbesondere ein oder zwei, gleiche oder verschiedene Heteroatome enthalten. Soweit nicht anders angegeben, können die Heterocyclen monocyclisch oder bicyclisch sein. Bevorzugt sind sie monocyclisch. Die Ringe enthalten bevorzugt 5, 6 oder 7 Ringglieder. Beispiele für monocyclische und bicyclische heterocyclische Systeme, von denen sich in den Verbindungen der Formel I vorkommende Reste ableiten können, sind Pyrrol, Furan; Thiophen, Imidazol, Pyrazol, 1,2,3-Triazol, 1,2,4-Triazol, 1,3-Dioxol, 1,3-Oxazol, 1,2-Oxazol, 1,3-Thiazol, 1,2-Thiazol, Tetrazol, Pyridin, Pyridazin, Pyrimidin, Pyrazin, Pyran, Thiopyran, 1,4-Dioxin, 1,2-Oxazin, 1,3-Oxazin, 1,4-Oxazin, 1,2-Thiazin, 1,3-Thiazin, 1,4-Thiazin, 1,2,3-Triazin, 1,2,4-Triazin, 1,3,5-Triazin, 1,2,4,5-Tetrazin, Azepin, 1,2-Diazepin, 1,3-Diazepin, 1,4-Diazepin, 1,3-Oxazepin, 1,3-Thiazepin, Indol, Benzothiophen, Benzofuran, Benzothiazol, Benzimidazol, Chinolin, Isochinolin, Cinnolin, Chinazolin, Chinoxalin, Phthalazin, Thienothiophene, 1,8-Naphthyridin und andere Naphthyridine, Pteridin oder Phenothiazin, alle jeweils in gesättigter Form (Perhydro-Form) oder in teilweise ungesättigter Form (zum Beispiel Dihydro-Form und Tetrahydro-Form) oder in maximal ungesättigter Form, soweit die betreffenden Formen bekannt und stabil sind. Zu den in Betracht kommenden Heterocyclen gehören beispielsweise auch die gesättigten Heterocyclen Pyrrolidin, Piperidin, Perhydroazepin (Hexamethylenimin), Piperazin, Morpholin, 1,3-Thiazolidin und Thiomorpholin, die - soweit dies in Einklang mit der jeweiligen Definition ist - Beispiele sind für Reste von gesättigten heterocyclischen Ringen und für Reste von Ringen, die von zwei an ein Stickstoffatom gebundenen Gruppen zusammen mit diesem Stickstoffatom gebildet werden. Der Sättigungsgrad von heterocyclischen Gruppen ist bei den einzelnen Definitionen angegeben. Ungesättigte Heterocyclen können zum Beispiel eine, zwei oder drei Doppelbindungen im Ring enthalten, und 5-Ringe und 6-Ringe in monocyclischen und bicyclischen Heterocyclen können insbesondere auch aromatisch sein.

Heterocyclische Reste können über jedes geeignete Ringkohlenstoffatom gebunden sein. Stickstoffheterocyclen, zum Beispiel Pyrrol, Imidazol, Pyrrolidin, Piperidin, Hexamethylenimin, 1,3-Thiazolidin, Morpholin, Thiomorpholin, Piperazin etc., können auch über jedes geeignete Ringstickstoffatom gebunden sein, insbesondere, wenn der Stickstoffheterocyclus an ein Kohlenstoffatom gebunden ist. Beispielsweise kann ein Thienylrest als 2-Thienylrest oder 3-Thienylrest vorliegen, ein Furylrest als 2-Furylrest oder 3-Furylrest, ein Piperidinrest als 1-Piperidinylrest (= Piperidinorest), 2-Piperidinylrest, 3-Piperidinylrest oder 4-Piperidinylrest, ein (Thio)Morpholinrest als 2-(Thio)Morpholinylrest, 3-(Thio)Morpholinylrest oder 4-(Thio)Morpholinylrest (= (Thio)Morpholinorest). Ein Rest, der sich vom 1,3-Thiazol ableitet, kann über die 2-Position, die 3-Position, die 4-Position oder die 5-Position gebunden sein, ein Rest, der sich vom Imidazol ableitet, über die 1-Position, die 2-Position, die 4-Position oder die 5-Position. Ein Pyridylrest kann ein 2-Pyridylrest, 3-Pyridylrest oder 4-Pyridylrest sein.

Soweit nicht anders angegeben, können die heterocyclischen Gruppen unsubstituiert sein oder einen oder mehrere, zum Beispiel einen, zwei, drei oder vier, insbesondere einen oder zwei, gleiche oder verschiedene Substituenten tragen. Die Substituenten in Heterocyclen können sich in beliebigen Positionen befinden, beispielsweise in einem 2-Thienylrest oder 2-Furylrest in der 3-Position und/oder in der 4-Position und/oder in der 5-Position, in einem 3-Thienylrest oder 3-Furylrest in der 2-Position und/oder in der 4-Position und/oder in der 5-Position, in einem 2-Pyridylrest in der 3-Position und/oder in der 4-Position und/oder in der 5-Position und/oder in der 6-Position, in einem 3-Pyridylrest in der 2-Position und/oder in der 4-Position und/oder in der 5-Position und/oder in der 6-Position, in einem 4-Pyridylrest in der 2-Position und/oder in der 3-Position und/oder in der 5-Position und/oder in der 6-Position. Soweit nicht anders angegeben, können als Substituenten zum Beispiel die in der Definition der Gruppe Aryl angegebenen Substituenten auftreten. Soweit nicht anders angegeben, kommen als Substituenten an einem substituierbaren Stickstoffatom eines Heterocyclus zum Beispiel unsubstituierte und substituierte ($C_1$-$C_4$)-Alkylreste, Arylreste oder die Reste -CO-($C_1$-$C_4$)-Alkyl, -$SO_2$-($C_1$-$C_4$)-Alkyl oder -$SO_2$-Aryl in Betracht. Geeignete Schwefelheterocyclen können auch als S-Oxide oder S,S-Dioxide vorliegen, das heißt an Stelle eines Schwefelatoms die Gruppe S(=O) oder die Gruppe $S(=O)_2$ enthalten. Geeignete Stickstoffatome in den Verbindungen der Formel I können auch als N-Oxide vorliegen. Pyridylreste können als Pyridin-N-oxide vorliegen.

Halogen bedeutet Fluor, Chlor, Brom oder Iod, vorzugsweise Fluor oder Chlor.

Die vorliegende Erfindung umfaßt alle stereoisomeren Formen der Verbindungen der Formel I. In den Verbindungen der Formel I enthaltene Asymmetriezentren können alle unabhängig voneinander die S-Konfiguration oder die R-Konfiguration aufweisen. Zur Erfindung gehören alle möglichen Enantiomeren und Diastereomeren, ebenso Mischungen von zwei oder mehr stereoisomeren Formen, zum Beispiel Mischungen von Enantiomeren und/oder Diastereomeren, in allen Verhältnissen. Enantiomere sind also in enantiomerenreiner Form, sowohl als linksdrehende als auch als rechtsdrehende Antipoden, in Form von Racematen und in Form von Mischungen der beiden Enantiomeren in allen Verhältnissen Gegenstand der Erfindung. Bei Vorliegen einer cis/trans-Isomerie, zum Beispiel an Cycloalkylgruppen, sind sowohl die cis-Form als auch die trans-Form und Gemische dieser Formen in allen Verhältnissen Gegenstand der Erfindung. Die Herstellung von einzelnen Stereoisomeren kann gewünschtenfalls durch Auftrennung eines Gemisches nach üblichen Methoden, zum Beispiel durch Chromatographie oder Kristallisation, durch Verwendung von stereochemisch einheitlichen Ausgangssubstanzen bei der Synthese oder durch stereoselektive Synthese erfolgen. Gegebenenfalls kann vor einer Trennung von Stereoisomeren eine Derivatisierung erfolgen. Die Trennung eines Stereoisomerengemisches kann auf der Stufe der Verbindungen der Formel 1 erfolgen oder auf der Stufe eines Zwischenprodukts im Verlaufe der Synthese. Bei Vorliegen von beweglichen Wasserstoffatomen umfaßt die vorliegende Erfindung auch alle tautomeren Formen der Verbindungen der Formel I.

Enthalten die Verbindungen der Formel I eine oder mehrere saure oder basische Gruppen, so sind auch die entsprechenden physiologisch oder toxikologisch verträglichen Salze Gegenstand der Erfindung, insbesondere die pharmazeutisch verwendbaren Salze. So können die Verbindungen der Formel 1, die saure Gruppen enthalten, an diesen Gruppen beispielsweise als Alkalimetallsalze, Erdalkalimetallsalze oder als Ammoniumsalze vorliegen und erfindungsgemäß verwendet werden. Beispiele für solche Salze sind Natriumsalze, Kaliumsalze, Calciumsalze, Magnesiumsalze, Salze mit Ammoniak oder organischen Aminen wie beispielsweise Ethylamin, Ethanolamin, Triethanolamin oder Aminosäuren, oder Salze, die quartäre organische Ammoniumionen als Kation enthalten. Verbindungen der Formel I, die eine oder mehrere basische, das heißt protonierbare, Gruppen enthalten, können in Form ihrer Säureadditionssalze mit physiologisch verträglichen anorganischen oder organischen Säuren vorliegen und erfindungsgemäß verwendet werden, zum Beispiel als Salze mit Chlorwasserstoff, Bromwasserstoff, Phosphorsäure, Schwefelsäure, Salpetersäure, Methansulfonsäure, p-Toluolsulfonsäure, Naphthalindisulfonsäuren, Oxalsäure, Essigsäure, Weinsäure, Milchsäure, Salicylsäure, Benzoesäure, Ameisensäure, Propionsäure, Pivalinsäure, Diethylessigsäure, Malonsäure, Bernsteinsäure, Pimelinsäure, Fumarsäure, Maleinsäure, Äpfelsäure, Sulfaminsäure, Phenylpropionsäure, Gluconsäure, Ascorbinsäure, Isonicotinsäure, Zitronensäure, Adipinsäure usw. Enthalten die Verbindungen der Formel I gleichzeitig saure und basische Gruppen im Molekül, so gehören neben den geschilderten Salzformen auch innere Salze oder Betaine (Zwitterionen) zu der Erfindung. Salze können aus den Verbindungen der Formel I nach üblichen, dem Fachmann bekannten Verfahren erhalten werden, beispielsweise durch Vereinigung mit einer organischen oder anorganischen Säure oder Base in einem Lösungsmittel oder Dispergiermittel, oder auch durch Anionenaustausch oder Kationenaustausch aus anderen Salzen. Die vorliegende Erfindung umfaßt auch alle Salze der Verbindungen der Formel I, die sich wegen geringer physiologischer Verträglichkeit nicht direkt für eine Verwendung in Arzneimitteln eignen, aber zum Beispiel als Zwischenprodukte für chemische Reaktionen oder für die Herstellung physiologisch verträglicher Salze in Betracht kommen.

Die vorliegende Erfindung umfaßt weiterhin alle Solvate von Verbindungen der Formel I, zum Beispiel Hydrate oder Addukte mit Alkoholen, sowie Derivate der Verbindungen der Formel I wie zum Beispiel Ester, und Pro-Drugs und aktive Metabolite.

Bevorzugt steht einer der Reste $R^1$ und $R^2$ für ($C_1$-$C_8$)-Alkyl, das durch einen oder mehrere gleiche oder verschiedene Substituenten aus der Reihe Hydroxy, ($C_1$-$C_4$)-Alkoxy, ($C_1$-$C_4$)-Alkyl-$S(O)_m$-, unsubstituiertes oder substituiertes Phenyl und unsubstituiertes oder substituiertes Naphthyl substituiert sein kann, oder für ($C_3$-$C_9$)-Cycloalkyl, das durch einen oder mehrere gleiche oder verschiedene Substituenten aus der Reihe ($C_1$-$C_4$)-Alkyl, Hydroxy, Amino und unsubstituiertes oder substituiertes Benzyl substituiert sein kann. Besonders bevorzugt steht der andere der Reste $R^1$ und $R^2$ für Wasserstoff, ($C_1$-$C_8$)-Alkyl, das durch einen oder mehrere gleiche oder verschiedene Substituenten aus der Reihe Hydroxy, ($C_1$-$C_4$)-Alkoxy, ($C_1$-$C_4$)-Alkyl-$S(O)_m$-, unsubstituiertes oder substituiertes Phenyl und unsubstituiertes oder substituiertes Naphthyl substituiert sein kann, oder für ($C_3$-$C_8$)-Cycloalkyl, das durch einen oder mehrere gleiche oder verschiedene Substituenten aus der Reihe ($C_1$-$C_4$)-Alkyl, Hydroxy, Amino und unsubstituiertes oder substituiertes Benzyl substituiert sein kann. Ganz besonders bevorzugt ist es, wenn einer der Reste $R^1$ und $R^2$ für ($C_1$-$C_8$)-Alkyl oder ($C_3$-$C_9$)-Cycloalkyl steht und der andere der Reste $R^1$ und $R^2$ für Wasserstoff steht, oder wenn die Reste $R^1$ und $R^2$ für gleiches oder verschiedenes ($C_1$-$C_8$)-Alkyl stehen, wobei alle Alkylreste und Cycloalkylreste unsubstituiert oder wie angegeben substituiert sein können. Speziell bevorzugt ist, wenn einer der Reste $R^1$ und $R^2$ für ($C_3$-$C_9$)-Cycloalkyl steht, das durch einen oder mehrere gleiche oder verschiedene Substituenten aus der Reihe ($C_1$-$C_4$)-Alkyl, Hydroxy, Amino und unsubstituiertes oder substituiertes Benzyl substituiert sein kann, und der andere der Reste $R^1$ und $R^2$ für Wasserstoff steht. Wenn einer der Reste $R^1$ und $R^2$ für ($C_3$-$C_9$)-Cycloalkyl steht, das durch einen oder mehrere gleiche oder verschiedene Substituenten aus der Reihe ($C_1$-$C_4$)-Alkyl, Hydroxy, Amino und unsubstituiertes oder substituiertes Benzyl substituiert sein kann, oder für den Rest eines 5-gliedrigen bis 7-gliedrigen gesättigten heterocyclischen Ringes steht, der ein oder zwei gleiche oder verschiedene Hetero-Ringglieder aus der Reihe O, $NR^{10}$ und $S(O)_m$ enthält und der durch einen oder mehrere gleiche oder verschiedene Substituenten aus der Reihe ($C_1$-$C_4$)-Alkyl, Hydroxy und Aryl-($C_1$-$C_4$)-alkylsubstituiert sein kann, dann steht der andere der Reste $R^1$ und $R^2$ bevorzugt für Wasserstoff.

Ein für $R^1$ oder $R^2$ stehender Alkylrest ist bevorzugt ein unsubstituierter oder substituierter ($C_1$-$C_4$)-Alkylrest. Ein für $R^1$ oder $R^2$ stehender ($C_3$-$C_9$)-Cycloalkylrest ist bevorzugt ein unsubstituierter oder substituierter Rest aus der Reihe Cyclopropyl, Cyclobutyl, Cyclopentyl, Cyclohexyl und Cycloheptyl, insbesondere unsubstituiertes oder substituiertes Cyclopentyl oder Cyclohexyl. Bevorzugt ist ein für $R^1$ oder $R^2$ stehender substituierter Cycloalkylrest durch einen oder mehrere Reste aus der Reihe ($C_1$-$C_4$)-Alkyl, Hydroxy und Amino substituiert, besonders bevorzugt durch einen oder mehrere ($C_1$-$C_4$)-Alkylreste oder durch eine Hydroxygruppe oder durch eine Aminogruppe, insbesondere durch eine Hydroxygruppe. Ein für $R^1$ oder $R^2$ stehender Rest eines 5-gliedrigen bis 7-gliedrigen gesättigten, heterocyclischen Ringes enthält bevorzugt ein Hetero-Ringglied aus der Reihe O, $NR^{10}$ und $S(O)_m$, besonders bevorzugt eine Gruppe $NR^{10}$ als Hetero-Ringglied. Bevorzugt ist ein derartiger heterocyclischer Ring über ein Ring-Kohlenstoffatom gebunden, das nicht direkt an ein Hetero-Ringglied gebunden ist. Beispiele für derartige Reste heterocyclischer Ringe sind gegebenenfalls substituiertes Pyrrolidinyl, zum Beispiel 3-Pyrrolidinyl, gegebenenfalls substituiertes Piperidinyl, zum Beispiel 3-Piperidinyl oder 4-Piperidinyl, Tetrahydrofuryl, zum Beispiel 3-Tetrahydrofuryl, Tetrahydrothienyl und dessen S-Oxide und S,S-Dioxide, zum Beispiel 3-Tetrahydrothienyl, oder Tetrahydro(thio)pyranyl.

Neben den vorstehend genannten bevorzugten Bedeutungen von $R^1$ und $R^2$ ist es weiterhin bevorzugt, wenn die Gruppe $R^1R^2N$ für einen über ein Ringstickstoffatom gebundenen Rest eines 5-gliedrigen, 6-gliedrigen oder 7-gliedrigen gesättigten heterocyclischen Ringes steht, der neben dem die Reste $R^1$ und $R^2$ tragenden Stickstoffatom als weiteres Hetero-Ringglied noch ein Sauerstoffatom oder eine Gruppe $S(O)_m$ enthalten kann und der durch einen oder mehrere gleiche oder verschiedene Substituenten aus der Reihe ($C_1$-$C_4$)-Alkyl, Hydroxy, ($C_1$-$C_4$)-Alkoxy und $R^{11}R^{12}N$ substituiert sein kann. Ein für $R^1R^2N$ stehender Rest eines heterocyclischen Ringes leitet sich bevorzugt von einem 5-gliedrigen oder 6-gliedrigen gesättigen heterocyclischen Ring ab, besonders bevorzugt vom Piperidin, Morpholin oder Thiomorpholin (und seinem S-Oxid und S,S-Dioxid), die wie angegeben substituiert sein können, ganz besonders bevorzugt vom unsubstituierten Piperidin, Morpholin oder Thiomorpholin (und seinem S-Oxid und S,S-Dioxid).

Die für $R^3$ stehende Arylgruppe steht bevorzugt für substituiertes Phenyl, ganz besonders bevorzugt für Phenyl, das durch einen oder zwei der oben in der Definition von Aryl angegebenen Substituenten substituiert ist. Speziell bevorzugt steht $R^3$ für Phenyl, das durch ein oder zwei Substituenten aus der Reihe Halogen und ($C_1$-$C_4$)-Alkyl substituiert ist, darüber hinaus bevorzugt für Phenyl, das durch Chlor oder Methyl substituiert ist. Der Substituent in einer für $R^3$ stehenden einfach substituierten Phenylgruppe steht bevorzugt in der para-Position.

Aryl steht bevorzugt für Phenyl oder 5-gliedriges oder 6-gliedriges monocyclisches Heteroaryl mit einem oder zwei, insbesondere einem, Heteroatom aus der Reihe N, O und S, das wie angegeben substituiert sein kann, besonders bevorzugt für unsubstituiertes oder substituiertes Phenyl oder unsubstituiertes Pyridyl, Thienyl oder Furyl, ganz besonders bevorzugt für unsubstituiertes oder substituiertes Phenyl oder unsubstituiertes Pyridyl.

Bevorzugte Verbindungen der Formel I sind solche, in denen einer oder mehrere der darin enthaltenen Reste bevorzugte Bedeutungen haben, wobei alle Kombinationen von bevorzugten Substituentendefinitionen und spezifischen bevorzugten Substituentenbedeutungen Gegenstand der vorliegenden Erfindung sind. Auch von allen bevorzugten Verbindungen der Formel I umfaßt die vorliegende Erfindung alle ihre stereoisomeren Formen und Mischungen davon in allen Verhältnissen, und ihre physiologisch verträglichen Salze.

Gegenstand der vorliegenden Erfindung sind auch Verfahren zur Herstellung der Verbindungen der Formel I, die im folgenden erläutert sind und nach denen die erfindungsgemäßen Verbindungen erhältlich sind. Die Herstellung der Verbindungen der Formel I kann erfolgen, indem zunächst in an sich bekannter Weise ein Amidin der Formel II mit einem 2-Oxocyclopentancarbonsäureester der Formel III zu einem 4-Hydroxycyclopentapyrimidin der Formel IV umgesetzt wird. R in der Formel III steht zum Beispiel für ($C_1$-$C_4$)-Alkyl wie Methyl oder Ethyl. Das Hydroxycyclopentapyrimidin der Formel IV wird anschließend aktiviert, zum Beispiel durch Überführung in ein 4-Halogencyclopentapyrimidin. Beispielsweise läßt sich die Verbindung der Formel IV durch Umsetzung mit einem Phosphorhalogenid wie Phosphoroxychlorid in das 4-Chlorcyclopentapyrimidin der Formel V überführen. Durch Umsetzung der Verbindung der Formel V (oder eines anderen reaktiven Derivats des Hydroxycyclopentapyrimidins) mit dem gewünschten Amin der Formel VI wird dann unter Austausch des Chlors gegen die Aminogruppe die erfindungsgemäße Verbindung der Formel I erhalten. Geeignete Lösungsmittel für diese Austauschreaktion sind zum Beispiel Wasser, Alkohole wie Methanol, Ethanol oder Isopropanol, Ether wie Tetrahydrofuran oder Dioxan, Amide wie Dimethylformamid (DMF) oder N-Methylpyrrolidon (NMP), oder Kohlenwasserstoffe oder halogenierte Kohlenwasserstoffe wie Benzol, Toluol, Xylol, Chlorbenzol oder Dichlorbenzol.

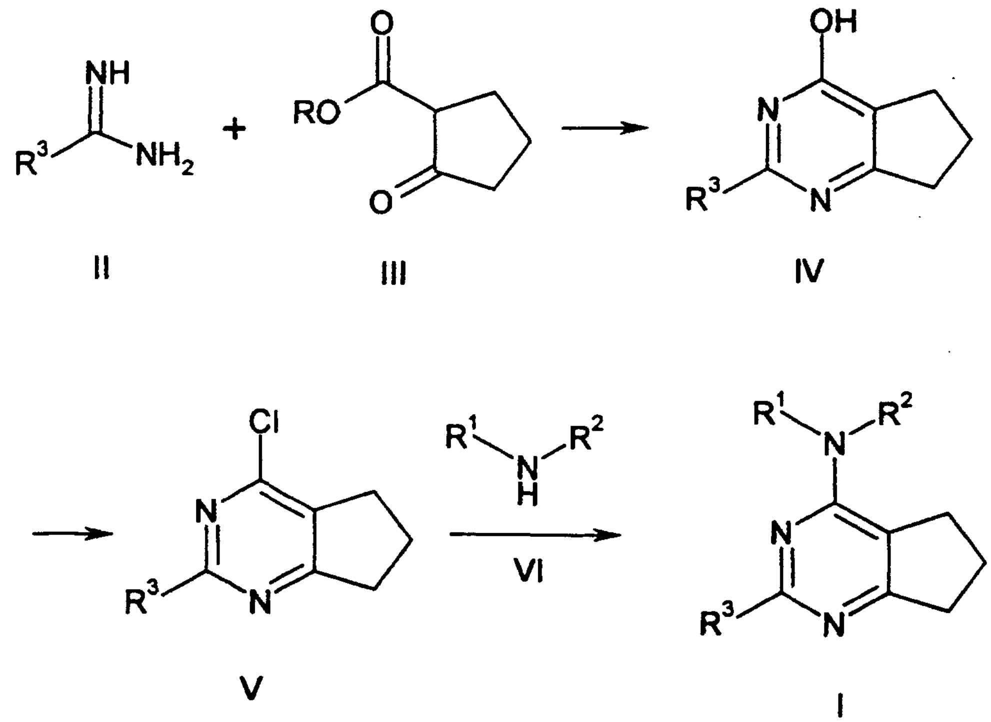

II III IV

V VI I

Diese Umsetzungen können in einem weiten Temperaturbereich durchgeführt werden. Bevorzugt sind Reaktionstemperaturen von 20 °C bis 150 °C. Sie können durch Zusatz von geeigneten Basen wie zum Beispiel Natriumbicarbonat, Natriumcarbonat, Kaliumcarbonat, Natriumalkoholaten, Triethylamin oder Pyridin beschleunigt werden, im ersten und im letzten Schritt zusätzlich auch durch überschüssiges Amidin bzw. Amin. An Stelle der freien Amidine der Formel II können auch die entsprechenden Amidiniumsalze eingesetzt werden. In diesem Falle ist es besonders günstig, den ersten Schritt unter Zusatz von Basen durchzuführen. Die Zwischenstufen der Formel IV und V und die Endverbindungen der Formel I können aus der jeweiligen Reaktionsmischung nach gängigen Verfahren wie Kristallisation, Sublimation, Chromatographie oder Destillation abgetrennt und gewünschtenfalls gereinigt werden, je nach den Umständen des Einzelfalles können die Zwischenstufen, aber auch ohne Isolierung weiter umgesetzt werden. In den so erhaltenen Verbindungen der Formel I können zudem funktionelle Gruppen umgewandelt werden. Beispielsweise können Thioethergruppierungen durch Oxidation mit einer Peroxyverbindung wie 3-Chlorperbenzoesäure oder Monoperoxyphthalsäure oder Wasserstoffperoxid in Sulfone oder Sulfoxide umgewandelt werden, oder es können Carbonsäureestergruppen zu den Carbonsäuren verseift werden.

Alle Reaktionen zur Synthese der Verbindungen der Formel I sind dem Fachmann an sich wohlbekannt und können unter Standardbedingungen nach oder analog zu Literaturvorschriften durchgeführt werden, wie sie zum Beispiel in Houben-Weyl, Methoden der Organischen Chemie, Thieme-Verlag, Stuttgart, oder Organic Reactions, John Wiley & Sons, New York, beschrieben sind. Je nach den Gegebenheiten des Einzelfalls kann es bei der Synthese der Verbindungen der Formel I zur Vermeidung von Nebenreaktionen auch vorteilhaft oder notwendig sein, bestimmte funktionelle Gruppen vorübergehend durch die Einführung von Schutzgruppen zu blockieren und später dann wieder freizusetzen, oder funktionelle Gruppen zunächst in Form von Vorstufen einzusetzen, aus denen in einem späteren Schritt dann die gewünschte funktionelle Gruppe erzeugt wird. Solche Synthesestrategien und die für den Einzelfall geeigneten Schutzgruppen oder Vorstufen sind dem Fachmann bekannt. Die Ausgangsamidine der Formel II oder deren Salze, die Oxoester der Formel III und die Amine der Formel VI sind kommerziell erhältlich oder können nach oder analog zu bekannten Verfahren hergestellt werden.

Die erfindungsgemäßen Verbindungen der Formel I bewirken über die Aktivierung der löslichen Guanylatcyclase (sGC) eine Erhöhung der cGMP-Konzentration und sind deshalb wertvolle Agenzien zur Therapie und Prophylaxe von Krankheiten, die mit einem niedrigen oder erniedrigten cGMP-Spiegel verbunden sind oder durch einen solchen verursacht werden oder zu deren Therapie oder Prophylaxe eine Erhöhung des vorhandenen cGMP-Spiegels angestrebt wird. Die Aktivierung der sGC durch die Verbindungen der Formel I kann zum Beispiel in dem unten beschriebenen Aktivitätsassay untersucht werden. Bevorzugte Substanzen der Formel I sind solche, die in diesem Assay eine mindestens dreifache Aktivierung zeigen.

Krankheiten und pathologische Zustände, die mit einem niedrigen cGMP-Spiegel verbunden sind oder bei denen eine Erhöhung des cGMP-Spiegels angestrebt wird und zu deren Therapie und Prophylaxe Verbindungen der

Formel I eingesetzt werden können, sind zum Beispiel Herz-Kreislauf-Erkrankungen wie endotheliale Dysfunktion, diastolische Dysfunktion, Atherosklerose, Bluthochdruck, stabile und instabile Angina pectoris, Thrombosen, Restenosen, Myocardinfarkt, Schlaganfälle, Herzinsuffizienz oder Pulmonalhypertonie, oder zum Beispiel erektile Dysfunktion, Asthma bronchiale, chronische Niereninsuffizienz und Diabetes. Verbindungen der Formel I können darüber hinaus eingesetzt werden bei der Therapie der Leberzirrhose sowie zur Verbesserung einer eingeschränkten Lemfähigkeit oder Gedächtnisleistung.

Die Verbindungen der Formel I und ihre physiologisch verträglichen Salze können somit am Tier, bevorzugt am Säugetier, und insbesondere am Menschen als Arzneimittel für sich allein, in Mischungen untereinander oder in Form von pharmazeutischen Zubereitungen verwendet werden. Gegenstand der vorliegenden Erfindung sind daher auch die Verbindungen der Formel I und ihre physiologisch verträglichen Salze zur Verwendung als Arzneimittel, ihre Verwendung zur Normalisierung eines gestörten cGMP-Haushalts und insbesondere ihre Verwendung in der Therapie und Prophylaxe der oben genannten Krankheitsbilder, sowie ihre Verwendung zur Herstellung von Medikamenten dafür. Weiterhin sind Gegenstand der vorliegenden Erfindung pharmazeutische Präparate, die eine wirksame Dosis mindestens einer Verbindung der Formel I und/oder eines physiologisch verträglichen Salzes davon als aktiven Bestandteil und einen pharmazeutisch verträglichen Träger, das heißt einen oder mehrere pharmazeutisch verträgliche Trägerstoffe und/oder Zusatzstoffe (oder Hilfsstoffe), enthalten.

Die erfindungsgemäßen Arzneimittel können oral, zum Beispiel in Form von Pillen, Tabletten, Filmtabletten, Dragees, Granulaten, Hart- und Weichgelatinekapseln, wäßrigen, alkoholischen oder öligen Lösungen, Sirupen, Emulsionen oder Suspensionen, oder rektal, zum Beispiel in Form von Suppositorien, verabreicht werden. Die Verabreichung kann aber auch parenteral erfolgen, zum Beispiel subkutan, intramuskulär oder intravenös in Form von Injektionslösungen oder Infusionslösungen. Weitere in Betracht kommende Applikationsformen sind zum Beispiel die perkutane oder topische Applikation, zum Beispiel in Form von Salben, Tinkturen, Sprays oder transdermalen therapeutischen Systemen, oder die inhalative Applikation in Form von Nasalsprays oder Aerosolmischungen, oder zum Beispiel Mikrokapseln, Implantate oder Rods. Die bevorzugte Applikationsform hängt zum Beispiel von der zu behandelnden Krankheit und ihrer Stärke ab.

Die pharmazeutischen Präparate enthalten normalerweise ungefähr 0.1 bis 1000 mg, vorzugsweise 0.2 bis 500 mg, insbesondere 1 bis 200 mg, Wirkstoff der Formel I und/oder dessen physiologisch verträgliche Salze und einen oder mehrere pharmazeutisch einwandfreie Trägerstoffe und/oder Zusatzstoffe. Je nach Art des pharmazeutischen Präparats kann aber auch eine größere Wirkstoffmenge enthalten sein. Die Herstellung der pharmazeutischen Präparate kann in an sich bekannter Weise erfolgen. Dazu werden ein oder mehrere Verbindungen der Formel I und/oder ihre physiologisch verträglichen Salze zusammen mit einem oder mehreren festen oder flüssigen galenischen Trägerstoffen und/oder Zusatzstoffen und, wenn gewünscht, in Kombination mit anderen Arzneimittelwirkstoffen mit therapeutischer oder prophylaktischer Wirkung in eine geeignete Verabreichungsform bzw. Dosierungsform gebracht, die dann als Arzneimittel in der Humanmedizin oder Veterinärmedizin verwendet werden kann. Die pharmazeutischen Präparate enthalten normalerweise 0.5 bis 90 Gewichtsprozent der Verbindungen der Formel I und/oder ihrer physiologisch verträglichen Salze.

Für die Herstellung beispielsweise von Pillen, Tabletten, Dragees und Hartgelatinekapseln kann man Lactose, Stärke, zum Beispiel Maisstärke, oder Stärkederivate, Talk, Stearinsäure oder deren Salze, etc. verwenden. Trägerstoffe für Weichgelatinekapseln und Suppositorien sind zum Beispiel Fette, Wachse, halbfeste und flüssige Polyole, natürliche oder gehärtete Öle etc. Als Trägerstoffe für die Herstellung von Lösungen, zum Beispiel Injektionslösungen, oder von Emulsionen oder Sirupen eignen sich beispielsweise Wasser, physiologische Kochsalzlösung, Alkohole wie Ethanol, Glycerin, Polyole, Saccharose, Invertzucker, Glucose, Mannit, pflanzliche Öle etc. Die Verbindungen der Formel I und ihre physiologisch verträglichen Salze können auch lyophilisiert werden und die erhaltenen Lyophilisate zum Beispiel zur Herstellung von Injektionspräparaten oder Infusionspräparaten verwendet werden. Als Trägerstoffe für Mikrokapseln, Implantate oder Rods eignen sich zum Beispiel Mischpolymerisate aus Glykolsäure und Milchsäure.

Die pharmazeutischen Präparate können neben den Wirkstoffen und Trägerstoffen noch übliche Zusatzstoffe enthalten, zum Beispiel Füllstoffe, Spreng-, Binde-, Gleit-, Netz-, Stabilisierungs-, Emulgier-, Dispergier-, Konservierungs-, Süß-, Färbe-, Geschmacks-, Aromatisierungs-, Dickungs-, Verdünnungsmittel, Puffersubstanzen, Lösungsmittel, Lösungsvermittler, Mittel zur Erzielung eines Depoteffekts, Salze zur Veränderung des osmotischen Drucks, Überzugsmittel oder Antioxidantien.

Die Dosierung des zu verabreichenden Wirkstoffs der Formel I und/oder eines physiologisch verträglichen Salze davon hängt vom Einzelfall ab und ist wie üblich für eine optimale Wirkung den individuellen Gegebenheiten anzupassen. So hängt sie ab von der Art und Stärke der zu behandelnden Krankheit sowie von Geschlecht, Alter, Gewicht und individueller Ansprechbarkeit des zu behandelnden Menschen oder Tieres, von der Wirkstärke und Wirkdauer der eingesetzten Verbindungen, davon, ob akut oder chronisch therapiert wird oder Prophylaxe betrieben wird, oder davon, ob neben Verbindungen der Formel I weitere Wirkstoffe verabreicht werden. Im allgemeinen ist eine Tagesdosis von etwa 0.01 bis 100 mg/kg, vorzugsweise 0.1 bis 10 mg/kg, insbesondere 0.3 bis 5 mg/kg (jeweils mg pro kg Körpergewicht) bei Verabreichung an einen ca. 75 kg schweren Erwachsenen zur Erzielung der angestrebten Wirkung

angemessen. Die Tagesdosis kann in einer Einzeldosis verabreicht werden oder, insbesondere bei der Applikation größerer Mengen, in mehrere, zum Beispiel zwei, drei oder vier, Einzeldosen aufgeteilt werden. Gegebenenfalls kann es, je nach individuellem Verhalten, erforderlich werden, von der angegebenen Tagesdosis nach oben oder nach unten abzuweichen.

Die Verbindungen der Formel I aktivieren die lösliche Guanylatcyclase. Aufgrund dieser Eigenschaft können sie außer als Arzneimittelwirkstoffe in der Humanmedizin und Veterinärmedizin auch als wissenschaftliches Tool oder als Hilfsmittel für biochemische Untersuchungen eingesetzt werden, bei denen eine derartige Beeinflussung der Guanylatcyclase beabsichtigt ist, sowie für diagnostische Zwecke, zum Beispiel in der in vitro-Diagnostik von Zellproben oder Gewebeproben. Femer können die Verbindungen der Formel I und ihre Salze, wie bereits oben erwähnt, als Zwischenprodukte zur Herstellung weiterer Arzneimittelwirkstoffe dienen.

Die nachfolgenden Beispiele erläutern die Erfindung, ohne sie einzuschränken.

Beispiele

Beispiel 1

2-(4-Chlorphenyl)-4-hydroxy-6,7-dihydro-5H-cyclopenta[d]pyrimidin

7.1 g 2-Oxocyclopentan-1-carbonsäuremethylester und 9.6 g 4-Chlorbenzamidin-Hydrochlorid wurden in 50 ml Methanol vorgelegt. Unter Rühren wurden 5.6 g Kalium-tert-butylat zugegeben. Die Reaktionsmischung wurde 4 Stunden unter Rückfluß gerührt und danach auf Eiswasser gegossen. Das kristalline Produkt wurde abgesaugt, mit Wasser nachgewaschen und aus Dimethylformamid umkristallisiert. Ausbeute: 8.8 g. Schmp.: 282 °C.

Analog wurden hergestellt:

Beispiel 2

2-(3-Chlorphenyl)-4-hydroxy-6,7-dihydro-5H-cyclopenta[d]pyrimidin,- Schmp.: 255 °C.

Beispiel 3

2-(4-Methoxyphenyl)-4-hydroxy-6,7-dihydro-5H-cyclopenta[d]pyrimidin; Schmp.: 251 °C.

Beispiel 4

2-(3,4-Dimethoxyphenyl)-4-hydroxy-6,7-dihydro-5H-cyclopenta[d]pyrimidin; Schmp.: 283 °C.

Beispiel 5

2-(3,5-Dichlorphenyl)-4-hydroxy-6,7-dihydro-5H-cyclopenta[d]pyrimidin; Schmp.: 297 °C.

Beispiel 6

2-(4-Aminocarbonylphenyl)-4-hydroxy-6,7-dihydro-5H-cyclopenta[d]pyrimidin; Schmp.: >300 °C.

Beispiel 7

2-(4-Methylphenyl)-4-hydroxy-6,7-dihydro-5H-cyclopenta[d]pyrimidin; Schmp.: 273 °C.

Beispiel 8

2-(4-Chlorphenyl)-4-chlor-6,7-dihydro-5H-cyclopenta[d]pyrimidin

8.0 g 2-(4-Chlorphenyl)-4-hydroxy-6,7-dihydro-5H-cyclopenta[d]pyrimidin werden in 10 ml Phosphoroxychlorid auf 100 °C erwärmt. Nach 3 Stunden wurde die abgekühlte Lösung vorsichtig auf Eiswasser gegossen. Das kristalline Produkt wurde abgesaugt, mit Wasser gut nachgewaschen und im Vakuum bei Raumtemperatur getrocknet.
Ausbeute: 6.5 g. Schmp.: 145 °C.

Analog wurden hergestellt:

Beispiel 9

2-(3-Chlorphenyl)-4-chlor-6,7-dihydro-5H-cyclopenta[d]pyrimidin; Schmp.: 138 °C.

Beispiel 10

2-(3,5-Dichlorphenyl)-4-chlor-6,7-dihydro-5H-cyclopenta[d]pyrimidin; Schmp.: 165 °C.

Beispiel 11

2-(4-Methoxyphenyl)-4-chlor-6,7-dihydro-5H-cyclopenta[d]pyrimidin; Schmp.: 109 °C.

Beispiel 12

2-(3,4-Dimethoxyphenyl)-4-chlor-6,7-dihydro-5H-cyclopenta[d]pyrimidin; Schmp.: 152 °C.

Beispiel 13

2-(4-Methylphenyl)-4-chlor-6,7-dihydro-5H-cyclopenta[d]pyrimidin; Schmp.: 162 °C.

Beispiel 14

2-(4-Cyanphenyl)-4-chlor-6,7-dihydro-5H-cyclopenta[d]pyrimidin

Die Verbindung wurde ausgehend von 2-(4-Aminocarbonylphenyl)-4-hydroxy-6,7-dihydro-5H-cyclopenta[d]pyrimidin analog Beispiel 8 erhalten. Schmp.: 201 °C.

Beispiel 15

2-(4-Chlorphenyl)-4-cyclopentylamino-6,7-dihydro-5H-cyclopenta[d]pyrimidin 0.265 g 2-(4-Chlorphenyl)-4-chlor-6,7-dihydro-5H-cyclopenta[d]pyrimidin, 0.4 g Cyclopentylamin und 1 ml N-Methylpyrrolidon wurden im Ölbad auf 130 °C erhitzt. Nach 5 Stunden wurde mit 20 ml Wasser verdünnt und bei Raumtemperatur gerührt. Das ausgefallene Produkt wurde abgesaugt, mit Wasser gewaschen und im Vakuum bei 40 °C getrocknet. Ausbeute: 0.26 g. Schmp.: 167 °C.

Beispiel 16

2-(3,5-Dichlorphenyl)-4-(trans-4-hydroxycyclohexylamino)-6,7-dihydro-5H-cyclopenta[d]pyrimidin-methansulfonsäuresalz

0.15 g 2-(3,5-Dichlorphenyl)-4-chlor-6,7-dihydro-5H-cyclopenta[d]pyrimidin, 0.39 g trans-4-Aminocyclohexanol-hydrochlorid, 0.18 g Kalium-tert-butylat und 1.5 ml N-Methylpyrrolidon wurden 2 Stunden bei 130 °C im Ölbad erhitzt. Zur abgekühlten Lösung wurden 15 ml Wasser gegeben und das ausgefallene Produkt abgesaugt. Der getrocknete Feststoff wurde in 8 ml Ethylacetat und 2 ml Isopropanol aufgenommen und mit Methansulfonsäure versetzt. Das ausgefallene Produkt wurde abgesaugt, mit Ethylacetat gewaschen und bei 40 °C im Vakuum getrocknet. Ausbeute: 0.12 g. Schmp.: 218 °C.

Analog den Beispielen 15 und 16 wurden die folgenden Verbindungen der Formel I hergestellt:

Beispiel 17

2-(4-Chlorphenyl)-4-(N-benzylpiperidin-4-ylamino)-6,7-dihydro-5H-cyclopenta[d]pyrimidin-dihydrochlorid; Schmp.: 275

°C.

Beispiel 18

2-(4-Chlorphenyl)-4-(2-hydroxyethylamino)-6,7-dihydro-5H-cyclopenta[d]pyrimidin; Schmp.: 160 °C.

Beispiel 19

2-(4-Chlorphenyl)-4-((3-pyridylmethyl)amino)-6,7-dihydro-5H-cyctopenta[d]pyrimidin Schmp.: 209 °C.

Beispiel 20

2-(3-Chlorphenyl)-4-morpholino-6,7-dihydro-5H-cyclopenta[d]pyrimidin; Schmp.: 132 °C.

Beispiel 21

2-(3-Chlorphenyl)-4-(2-hydroxyethylamino)-6,7-dihydro-5H-cyclopenta[d]pyrimidin; Schmp.: 150 °C.

Beispiel 22

2-(3-Chlorphenyl)-4-((3-pyridylmethyl)amino)-6,7-dihydro-5H-cyclopenta[d]pyrimidin; Schmp.: 185 °C.

Beispiel 23

2-(3-Chlorphenyl)-4-(2-(3-methoxyphenyl)-ethylamino)-6,7-dihydro-5H-cyclopenta[d]pyrimidin-hydrochlorid; Schmp.: 192 °C.

Beispiel 24

2-(4-Chlorphenyl)-4-thiomorpholino-6,7-dihydro-5H-cyclopenta[d]pyrimidin; Schmp.: 159 °C.

Beispiel 25

2-(3-Chlorphenyl)-4-diethylamino-6,7-dihydro-5H-cyclopenta[d]pyrimidin-hydrochlorid; Schmp.: 185 °C.

Beispiel 26

2-(4-Chlorphenyl)-4-diethylamino-6,7-dihydro-5H-cyclopenta[d]pyrimidin-hydrochlorid; Schmp.: 185 °C.

Beispiel 27

2-(4-Chlorphenyl)-4-(2-(3-methoxyphenyl)-ethylamino)-6,7-dihydro-5H-cyclopenta[d]pyrimidin-hydrochlorid; Schmp.:

230 °C.

Beispiel 28

2-(4-Chlorphenyl)-4-(2-methoxyethylamino)-6,7-dihydro-5H-cyclopenta[d]pyrimidin-hydrochlorid; Schmp.: 208 °C.

Beispiel 29

2-(4-Chlorphenyl)-4-isobutylamino-6,7-dihydro-5H-cyclopenta[d]pyrimidin; Schmp.: 121 °C.

Beispiel 30

2-(4-Chlorphenyl)-4-butylamino-6,7-dihydro-5H-cyclopenta[d]pyrimidin; Schmp.: 121 °C.

Beispiel 31

2-(3-Chlorphenyl)-4-(2,2,6,6-tetramethylpiperidin-4-yl-amino)-6,7-dihydro-5H-cyclopenta[d]pyrimidin; Schmp.: 182 °C.

Beispiel 32

2-(3-Chlorphenyl)-4-cyclopentylamino-6,7-dihydro-5H-cyclopenta[d]pyrimidin; Schmp.: 129 °C.

Beispiel 33

2-(3-Chlorphenyl)-4-butylamino-6,7-dihydro-5H-cyclopenta[d]pyrimidin; Schmp.: Öl

Beispiel 34

2-(4-Chlorphenyl)-4-(trans-4-hydroxycyclohexylamino)-6,7-dihydro-5H-cyclopenta[d]pyrimidin; Schmp.: 216 °C.

Beispiel 35

2-(4-Chlorphenyl)-4-dipropylamino-6,7-dihydro-5H-cyclopenta[d]pyrimidin; Schmp.: 114 °C.

Beispiel 36

2-(3-Chlorphenyl)-4-pyrrolidino-6,7-dihydro-5H-cyclopenta[d]pyrimidin; Schmp.: 150 °C.

Beispiel 37

2-(3-Chlorphenyl)-4-hexamethylenimino-6,7-dihydro-5H-cyclopenta[d]pyrimidin-hydrochlorid; Schmp.: Harz

Beispiel 38

2-(3-Chlorphenyl)-4-thiomorpholino-6,7-dihydro-5H-cyclopenta[d]pyrimidin; Schmp.: 134 °C.

Beispiel 39

2-(4-Methylphenyl)-4-(2-hydroxyethylamino)-6,7-dihydro-5H-cyclopenta[d]pyrimidin; Schmp.: 191 °C.

Beispiel 40

2-(4-Methylphenyl)-4-butylamino-6,7-dihydro-5H-cyclopenta[d]pyrimidin; Schmp.: 133°C.

Beispiel 41

2-(4-Methylphenyl)-4-cyclopentylamino-6,7-dihydro-5H-cyclopenta[d]pyrimidin; Schmp.: 183 °C.

Beispiel 42

2-(4-Methylphenyl)-4-cyclohexylamino-6,7-dihydro-5H-cyclopenta[d]pyrimidin; Schmp.: 163 °C.

Beispiel 43

2-(4-Methylphenyl)-4-(trans-4-hydroxycyclohexylamino)-6,7-dihydro-5H-cyclopenta[d]pyrimidin; Schmp.: 191 °C.

Beispiel 44

2-(4-Methylphenyl)-4-((3-pyridylmethyl)amino)-6,7-dihydro-5H-cyclopenta[d]pyrimidin; Schmp.: 225 °C.

Beispiel 45

2-(4-Methylphenyl)-4-dipropylamino-6,7-dihydro-5H-cyclopenta[d]pyrimidin; Schmp.: 117 °C.

Beispiel 46

2-(4-Methylphenyl)-4-pyrrolidino-6,7-dihydro-5H-cyclopenta[d]pyrimidin; Schmp.: 203 °C.

Beispiel 47

2-(4-Methytphenyl)-4-thiomorpholino-6,7-dihydro-5H-cyclopenta[d]pyrimidin; Schmp.: 160 °C.

Beispiel 48

2-(3,5-Dichlorphenyl)-4-(4-hydroxybutylamino)-6,7-dihydro-5H-cyclopenta[d]pyrimidin; Schmp.: 142 °C.

Beispiel 49

2-(3,5-Dichlorphenyl)-4-butylamino-6,7-dihydro-5H-cyclopenta[d]pyrimidin; Schmp.: 93 °C.

Beispiel 50

2-(3,5-Dichlorphenyl)-4-cyclopentylamino-6,7-dihydro-5H-cyclopenta[d]pyrimidin; Schmp.: 153 °C.

Beispiel 51

2-(3,5-Dichlorphenyl)-4-((3-pyridylmethyl)amino)-6,7-dihydro-5H-cyclopenta[d]pyrimidin; Schmp.: 207 °C.

Beispiel 52

2-(3,5-Dichlorphenyl)-4-dipropylamino-6,7-dihydro-5H-cyclopenta[d]pyrimidin; Schmp.: 120 °C.

Beispiel 53

2-(3,5-Dichlorphenyl)-4-piperidino-6,7-dihydro-5H-cyclopenta[d]pyrimidin; Schmp.: 147 °C.

Beispiel 54

2-(4-Methoxyphenyl)-4-(3-hydroxypropylamino)-6,7-dihydro-5H-cyclopenta[d]pyrimidin; Schmp.: 165 °C.

Beispiel 55

2-(4-Methoxyphenyl)-4-butylamino-6,7-dihydro-5H-cyclopenta[d]pyrimidin; Schmp.: 119 °C.

Beispiel 56

2-(4-Methoxyphenyl)-4-cyclopentylamino-6,7-dihydro-5H-cyclopenta[d]pyrimidin; Schmp.: 154 °C.

Beispiel 57

2-(4-Methoxyphenyl)-4-cyclohexylamino-6,7-dihydro-5H-cyclopenta[d]pyrimidin; Schmp.: 138 °C.

Beispiel 58

2-(4-Methoxyphenyl)-4-(2-(2-chlorphenyl)-ethylamino)-6,7-dihydro-5H-cyclopenta[d]pyrimidin; Schmp.: 145 °C.

Beispiel 59

2-(4-Methoxyphenyl)-4-dipropylamino-6,7-dihydro-5H-cyclopenta[d]pyrimidin; Schmp.: 74 °C.

Beispiel 60

2-(4-Methoxyphenyl)-4-hexamethylenimino-6,7-dihydro-5H-cyclopenta[d]pyrimidin; Schmp.: 99 °C.

Beispiel 61

2-(4-Methoxyphenyl)-4-morpholino-6,7-dihydro-5H-cyclopenta[d]pyrimidin; Schmp.: 192 °C.

Beispiel 62

2-(3,4-Dimethoxyphenyl)-4-(3-methoxypropylamino)-6,7-dihydro-5H-cyclopenta[d]pyrimidin; Schmp.: 107 °C.

Beispiel 63

2-(3,4-Dimethoxyphenyl)-4-cyclopentylamino-6,7-dihydro-5H-cyclopenta[d]pyrimidin; Schmp.: 138 °C.

Beispiel 64

2-(3,4-Dimethoxyphenyl)-4-cyclohexylamino-6,7-dihydro-5H-cyclopenta[d]pyrimidin; Schmp.: 70 °C.

Beispiel 65

2-(3,4-Dimethoxyphenyl)-4-butylamino-6,7-dihydro-5H-cyclopenta[d]pyrimidin; Schmp.: 115 °C.

Beispiel 66

2-(3,4-Dimethoxyphenyl)-4-dipropylamino-6,7-dihydro-5H-cyclopenta[d]pyrimidin; Schmp.: 107 °C.

Beispiel 67

2-(3,4-Dimethoxyphenyl)-4-(2,6-dimethylmorpholino)-6,7-dihydro-5H-cyclopenta[d]pyrimidin; Schmp.: 142 °C.

Beispiel 68

2-(3,4-Dimethoxyphenyl)-4-(di-(2-hydroxyethyl)amino)-6,7-dihydro-5H-cyclopenta[d]pyrimidin; Schmp.: 115 °C.

Beispiel 69

2-(4-Cyanphenyl)-4-(3-methoxypropylamino)-6,7-dihydro-5H-cyclopenta[d]pyrimidin; Schmp.: 130 °C.

Beispiel 70

2-(4-Cyanphenyl)-4-benzylamino-6,7-dihydro-5H-cyclopenta[d]pyrimidin; Schmp.: 168 °C.

Beispiel 71

2-(4-Cyanphenyl)-4-diethylamino-6,7-dihydro-5H-cyclopenta[d]pyrimidin; Schmp.: 130 °C.

Beispiel 72

2-(4-Cyanphenyl)-4-piperidino-6,7-dihydro-5H-cyclopenta[d]pyrimidin; Schmp.: 152 °C.

Beispiel 73

2-(4-Cyanphenyl)-4-morpholino-6,7-dihydro-5H-cyclopenta[d]pyrimidin; Schmp.: 247 °C.

Beispiel 74

2-(4-Cyanphenyl)-4-((3-pyridylmethyl)amino)-6,7-dihydro-5H-cyclopenta[d]pyrimidin; Schmp.: 219 °C.

Pharmakologische Untersuchungen

Aktivierung der löslichen Guanylatcyclase

Die Aktivierung der löslichen Guanylatcyclase (sGC), die die Umwandlung von Guanosintriphosphat (GTP) in cyclisches Guanosinmonophosphat (cGMP) und Pyrophosphat katalysiert, durch die erfindungsgemäßen Verbindungen wurde mit Hilfe eines Enzym-Immuno-Assays (EIA) der Firma Amersham quantifiziert. Dazu wurden die Prüfsubstanzen zunächst mit sGC in Mikrotiterplatten inkubiert und dann die Menge des entstandenen cGMP bestimmt.

Die eingesetzte sGC war aus Rinderlunge isoliert worden (siehe Methods in Enzymology, Band 195, S. 377). Die Testlösungen (100 µl pro well) enthielten 50 mM Triethanolamin(TEA)-Puffer (pH 7.5), 3 mM $MgCl_2$, 3 mM reduziertes Glutathion (GSH), 0.1 mM GTP, 1 mM 3-Isobutyl-1-methylxanthin (IBMX), geeignet verdünnte Enzymlösung sowie die Prüfsubstanz bzw. bei den Kontrollversuchen Lösungsmittel. Die Prüfsubstanzen wurden in Dimethylsulfoxid (DMSO) gelöst und die Lösung mit DMSO/Wasser verdünnt, so daß die Endkonzentration c an Prüfsubstanz im Testansatz 50 µM betrug. Die DMSO-Konzentration im Testansatz betrug 5 % (v/v).

Die Reaktion wurde durch Zugabe der sGC gestartet. Der Reaktionsmix wurde für 15 bis 20 Minuten bei 37 °C inkubiert und dann durch Eiskühlung und Zugabe des Stop-Reagenz (50 mM EDTA, pH 8.0) gestoppt. Ein Aliquot von 50 µl wurde entnommen und zur Bestimmung des cGMP-Gehaltes mit dem Acetylierungs-Protokoll des Amersham-cGMP-EIA-Kits eingesetzt. Die Absorption der Proben wurde bei 450 nm (Referenzwellenlänge 620 nm) in einem Mikrotiterplatten-Lesegerät gemessen. Die cGMP-Konzentration wurde über eine Eichkurve ermittelt, die unter denselben Versuchsbedingungen erhalten wurde. Die Aktivierung der sGC durch eine Prüfsubstanz wird angegeben als n-fache Stimulation der basalen Enzymaktivität, die bei den Kontrollversuchen (mit Lösungsmittel statt Prüfsubstanz) gefunden wurde, berechnet nach der Formel

$$\text{n-fache Stimulierung} = [\text{cGMP}]_{\text{Prüfsubstanz}} / [\text{cGMP}]_{\text{Kontrolle}}.$$

Es wurden folgende Ergebnisse erhalten:

| Verbindung des Beispiels Nr. | n-fache Stimulation bei c = 50 µM |
|---|---|
| 15 | 7 |
| 16 | 34 |
| 25 | 6 |
| 26 | 5 |
| 30 | 6 |
| 32 | 15 |
| 33 | 5 |
| 34 | 35 |
| 35 | 7 |
| 40 | 8 |
| 41 | 9 |

Tabelle fortgesetzt

| Verbindung des Beispiels Nr. | n-fache Stimulation bei c = 50 μM |
|---|---|
| 42 | 10 |
| 43 | 23 |
| 44 | 7 |
| 45 | 7 |
| 48 | 9 |
| 49 | 5 |
| 50 | 8 |

**Patentansprüche**

**1.** Verbindungen der Formel I,

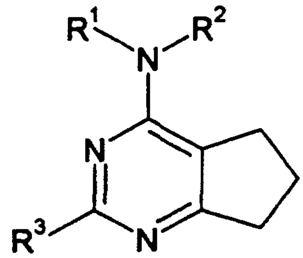

I

in der

$R^1$ und $R^2$, die unabhängig voneinander sind und gleich oder verschieden sein können, für Wasserstoff stehen, oder für $(C_1-C_8)$-Alkyl stehen, das durch einen oder mehrere gleiche oder verschiedene Substituenten aus der Reihe Hydroxy, $(C_1-C_4)$-Alkoxy, $(C_1-C_4)$-Alkyl-$S(O)_m$-, Phenyl, Naphthyl und Pyridyl substituiert sein kann, oder für $(C_3-C_9)$-Cyctoalkyl stehen, das durch einen oder mehrere gleiche oder verschiedene Substituenten aus der Reihe $(C_1-C_4)$-Alkyl, Hydroxy, Amino und Benzyl substituiert sein kann, oder für den Rest eines 5-gliedrigen bis 7-gliedrigen gesättigten heterocyclischen Ringes stehen, der ein oder zwei gleiche oder verschiedene Hetero-Ringglieder aus der Reihe O, $NR^{10}$ und $S(O)_m$ enthält und der durch einen oder mehrere gleiche oder verschiedene Substituenten aus der Reihe $(C_1-C_4)$-Alkyl, Hydroxy und Aryl-$(C_1-C_4)$-alkyl- substituiert sein kann, wobei in den Resten $R^1$ oder $R^2$ enthaltene Reste Phenyl, Naphthyl, Pyridyl und Benzyl unsubstituiert sein können oder im aromatischen Ring durch einen oder mehrere gleiche oder verschiedene Substituenten aus der Reihe Halogen, $(C_1-C_4)$-Alkyl, Phenyl, $CF_3$, $NO_2$, OH, -O-$(C_1-C_4)$-Alkyl, -O-$(C_2-C_4)$-Alkyl-O-$(C_1-C_4)$-alkyl, $(C_1-C_2)$-Alkylendioxy, $NH_2$, -NH-$(C_1-C_4)$-Alkyl, -N($(C_1-C_4)$-Alkyl$)_2$, -NH-CHO, -NH-CO-$(C_1-C_4)$-Alkyl, -CN, -CO-$NH_2$, -CO-NH-$(C_1-C_4)$-Alkyl, -CO-N($(C_1-C_4)$-Alkyl$)_2$, -CO-OH, -CO-O-$(C_1-C_4)$-Alkyl, -CHO und -CO-$(C_1-C_4)$-Alkyl substituiert sein können,
wobei aber $R^1$ und $R^2$ nicht beide gleichzeitig für Wasserstoff stehen können;
oder
der Rest $R^1R^2N$ für einen über ein Ringstickstoffatom gebundenen Rest eines 5-gliedrigen bis 7-gliedrigen gesättigten heterocyclischen Ringes steht, der neben dem die Reste $R^1$ und $R^2$ tragenden Stickstoffatom ein weiteres Hetero-Ringglied aus der Reihe O und $S(O)_m$ enthalten kann und der durch einen oder mehrere gleiche oder verschiedene Substituenten aus der Reihe $(C_1-C_4)$-Alkyl, Hydroxy, $(C_1-C_4)$-Alkoxy und $R^{11}R^{12}N$ substituiert sein kann;
$R^3$ für Aryl steht, aber nicht für unsubstituiertes Phenyl stehen kann;
$R^{10}$ für Wasserstoff, $(C_1-C_4)$-Alkyl, Aryl-$(C_1-C_4)$-alkyl-, Hydroxy-$(C_1-C_4)$-alkyl-, Hydroxycarbonyl-$(C_1-C_4)$-alkyl-, ($(C_1-C_4)$-Alkoxycarbonyl)-$(C_1-C_4)$-alkyl-, $R^{11}R^{12}N$-CO-$(C_1-C_4)$-alkyl-, $R^{13}$-$SO_2$- oder Aryl steht;
$R^{11}$ und $R^{12}$ für gleiche oder verschiedene Reste aus der Reihe Wasserstoff und $(C_1-C_4)$-Alkyl stehen;
$R^{13}$ für $(C_1-C_4)$-Alkyl, Aryl oder $R^{11}R^{12}N$ steht;
Aryl für Phenyl, Naphthyl oder Heteroaryl steht, die alle durch einen oder mehrere gleiche oder verschiedene

Substituenten aus der Reihe Halogen, $(C_1-C_4)$-Alkyl, Phenyl, $CF_3$, $NO_2$, OH, -O-$(C_1-C_4)$-Alkyl, -O-$(C_2-C_4)$-Alkyl-O-$(C_1-C_4)$-alkyl, $(C_1-C_2)$-Alkylendioxy, $NH_2$, -NH-$(C_1-C_4)$-Alkyl, -N($(C_1-C_4)$-Alkyl$)_2$, -NH-CHO, -NH-CO-$(C_1-C_4)$-Alkyl, -CN, -CO-$NH_2$, -CO-NH-$(C_1-C_4)$-Alkyl, -CO-N($(C_1-C_4)$-Alkyl$)_2$, -CO-OH, -CO-O-$(C_1-C_4)$-Alkyl, -CHO und -CO-$(C_1-C_4)$-Alkyl substituiert sein können;
Heteroaryl für den Rest eines monocyclischen 5-gliedrigen oder 6-gliedrigen aromatischen Heterocyclus oder eines bicyclischen 8-gliedrigen bis 10-gliedrigen aromatischen Heterocyclus steht, die jeweils ein oder mehrere gleiche oder verschiedene Ringheteroatome aus der Reihe N, O und S enthalten;
m für 0, 1 oder 2 steht;

wobei geeignette Stickstoffatome in den Verbindungen der Formel I auch als N-Oxide vorliegen können ;
in allen ihren stereoisomeren Formen une Mischungen davon in allen Verhältnissen, une ihre physiologisch verträglichen Salze.

**2.** Verbindungen der Formel I gemäß Anspruch 1, worin

einer der Reste $R^1$ und $R^2$ für $(C_1-C_8)$-Alkyl steht, das durch einen oder mehrere gleiche oder verschiedene Substituenten aus der Reihe Hydroxy, $(C_1-C_4$-Alkoxy, $(C_1-C_4)$-Alkyl-$S(O)_m$, unbustituiertes oder substituiertes Phenyl und unsubstituiertes oder substituiertes Naphthyl substituiert sein kann, oder für $(C_3-C_9)$-Cycloalkyl steht, das durch einen oder mehrere gleiche oder verschiedene Substituenten aus der Reihe $(C_1-C_4)$-Alkyl, Hydroxy, Amino und unsubstituiertes oder substituiertes Benzyl substituiert sein kann, und der andere der Reste $R^1$ und $R^2$ für Wasserstoff steht, oder für $(C_1-C_8$-Alkyl steht, das durch einen oder mehrere gleiche oder verschiedene Substituenten aus der Reihe Hydroxy, $(C_1-C_4$-Alkoxy, $(C_1-C_4$-Alkyl-$S(O)_m$, unsubstituiertes oder substituiertes Phenyl und unsubstituiertes oder substituiertes Naphthyl substituiert sein kann, oder für $(C_3-C_9)$-Cycloalkyl steht, das durch einen oder mehrere gleiche oder verschiedene Substituenten aus der Reihe $(C_1-C_4$-Alkyl, Hydroxy, Amino und unsubstituiertes oder substituiertes Benzyl substituiert sein kann;
oder
$R^1R^2N$ für einen über ein Ringstickstoffatom gebundenen Rest eines 5-gleidrigen, 6-gliedrigen oder 7-gliedrigen gesättigten heterocyclischen Ringes steht, der neben dem die Reste $R^1$ und $R^2$ tragenden Stickstoffatom als weiteres Hetero-Ringglied noch ein Sauerstoffatom oder eine Gruppe $S(O)_m$ enthalten kann und der durch einen oder mehrere gleiche oder verschiedene Substituenten aus der Reihe $(C_1-C_4)$-Alkyl, Hydroxy, $(C_1-C_4$-Alkoxy und $R^{11}R^{12}N$ substituiert sein kann; in allen ihren stereoisomeren Formen une Mischungen davon in allen Verhältnissen, und ihre physiologisch verträglichen Salze.

**3.** Verbindungen der Formel I gemäß Anspruch 1 und/oder 2, worin einer der Reste $R^1$ und $R^2$ für $(C_1-C_4)$-Alkyl steht, das durch einen oder mehrere gleiche oder verschiedene Substituenten aus der Reihe Hydroxy, $(C_1-C_4)$-Alkoxy, $(C_1-C_4)$-Alkyl-$S(O)_m$-, unsubstituiertes oder substituiertes Phenyl und unsubstituiertes oder substituiertes Naphthyl substituiert sein kann, oder für $(C_3-C_9)$-Cycloalkyl steht, das durch einen oder mehrere gleiche oder verschiedene Substituenten aus der Reihe $(C_1-C_4)$-Alkyl, Hydroxy, Amino und unsubstituiertes oder substituiertes Benzyl substituiert sein kann, und der andere der Reste $R^1$ und $R^2$ für Wasserstoff steht, oder die Reste $R^1$ und $R^2$ für gleiches oder verschiedenes $(C_1-C_4)$-Alkyl stehen, das durch einen oder mehrere gleiche oder verschiedene Substituenten aus der Reihe Hydroxy, $(C_1-C_4)$-Alkoxy, $(C_1-C_4)$-Alkyl-$S(O)_m$-, unsubstituiertes oder substituiertes Phenyl und unsubstituiertes oder substituiertes Naphthyl substituiert sein kann; in allen ihren stereoisomeren Formen und Mischungen davon in allen Verhältnissen, und ihre physiologisch verträglichen Salze.

**4.** Verbindungen der Formel I gemäß Anspruch 1 und/oder 2, worin einer der Reste $R^1$ und $R^2$ für $(C_1-C_8)$-Alkyl steht, das durch einen oder mehrere gleiche oder verschiedene Substituenten aus der Reihe Hydroxy, $(C_1-C_4)$-Alkoxy, $(C_1-C_4)$-Alkyl-$S(O)_m$-, unsubstituiertes oder substituiertes Phenyl und unsubstituiertes oder substituiertes Naphthyl substituiert sein kann, oder für $(C_3-C_9)$-Cycloalkyl steht, das durch einen oder mehrere gleiche oder verschiedene Substituenten aus der Reihe $(C_1-C_4)$-Alkyl, Hydroxy, Amino und unsubstituiertes oder substituiertes Benzyl substituiert sein kann; und der andere der Reste $R^1$ und $R^2$ für Wasserstoff steht; in allen ihren stereoisomeren Formen und Mischungen davon in allen Verhältnissen, und ihre physiologisch verträglichen Salze.

**5.** Verbindungen der Formel I gemäß einem oder mehreren der Ansprüche 1 bis 4, worin einer der Reste $R^1$ und $R^2$ für $(C_3-C_9)$-Cycloalkyl steht, das durch einen oder mehrere gleiche oder verschiedene Substituenten aus der Reihe $(C_1-C_4)$-Alkyl, Hydroxy, Amino und Benzyl substituiert sein kann; und der andere der Reste $R^1$ und $R^2$ für Wasserstoff steht;
in allen ihren stereoisomeren Formen und Mischungen davon in allen Verhältnissen, und ihre physiologisch verträglichen Salze.

6. Verbindungen der Formel I gemäß einem oder mehreren der Ansprüche 1 bis 5, worin ein für $R^1$ oder $R^2$ stehender ($C_3$-$C_9$)-Cycloalkylrest ein unsubstituierter oder substituierter Rest aus der Reihe Cyclopentyl und Cyclohexyl ist; in allen ihren stereoisomeren Formen und Mischungen davon in allen Verhältnissen, und ihre physiologisch verträglichen Salze.

7. Verbindungen der Formel I gemäß einem oder mehreren der Ansprüche 1 bis 6, worin ein für $R^1$ oder $R^2$ stehender substituierter Cycloalkylrest durch einen oder mehrere Reste aus der Reihe ($C_1$-$C_4$)-Alkyl, Hydroxy und Amino substituiert ist; in allen ihren stereoisomeren Formen und Mischungen davon in allen Verhältnissen, und ihre physiologisch verträglichen Salze.

8. Verbindungen der Formel I gemäß einem oder mehreren der Ansprüche 1 bis 7, worin ein substituierter Cycloalkylrest durch einen oder zwei gleiche oder verschiedene Substituenten substituiert ist; in allen ihren stereoisomeren Formen und Mischungen davon in allen Verhältnissen, und ihre physiologisch verträglichen Salze.

9. Verbindungen der Formel I gemäß einem oder mehreren der Ansprüche 1 bis 8, worin ein für $R^1$ oder $R^2$ stehender substituierter Cycloalkylrest durch eine Hydroxygruppe substituiert ist; in allen ihren stereoisomeren Formen und Mischungen davon in allen Verhältnissen, und ihre physiologisch verträglichen Salze.

10. Verbindungen der Formel I gemäß einem oder mehreren der Ansprüche 1 bis 9, worin ein substituierter Cycloalkylrest ein 4-Hydroxycyclohexylrest ist; in allen ihren stereoisomeren Formen und Mischungen davon in allen Verhältnissen, und ihre physiologisch verträglichen Salze.

11. Verbindungen der Formel I gemäß einem oder mehreren der Anspüche 1 bis 10, **dadurch gekennzeichnet gekennzeichnet**, daß sie aus den folgenden Verbindungen ausgewählt sind:

2-(4-Chlorphenyl)-4-cyclopentylamino-6,7-dihydro-5H-cyclopenta[d]pyrimidin,
2-(3,5-Dichlorphenyl)-4-(trans-4-hydroxycyclohexylamino)-6,7-dihydro-5H-cyclopenta[d]pyrimidin,
2-(3-Chlorphenyl)-4-cyclopentylamino-6,7-dihydro-5H-cyclopenta[d]pyrimidin,
2-(4-Chlorphenyl)-4-(trans-4-hydroxycyclohexylamino)-6,7-dihydro-5H-cyclopenta[d]pyrimidin,
2-(4-Methylphenyl)-4-cyclopentylamino-6,7-dihydro-5H-cyclopenta[d]pyrimidin,
2-(4-Methylphenyl)-4-cyclohexylamino-6,7-dihydro-5H-cyclopenta[d]pyrimidin,
2-(4-Methylphenyl)-4-(trans-4-hydroxycyclohexylamino)-6,7-dihydro-5H-cyclopenta[d]pyrimidin,
2-(3,5-Dichlorphenyl)-4-cyclopentylamino-6,7-dihydro-5H-cyclopenta[d]pyrimidin,
2-(4-Methoxyphenyl)-4-cyclopentylamino-6,7-dihydro-5H-cyclopenta[d]pyrimidin,
2-(4-Methoxyphenyl)-4-cyclohexylamino-6,7-dihydro-5H-cyclopenta[d]pyrimidin,
2-(3,4-Dimethoxyphenyl)-4-cyclopentylamino-6,7-dihydro-5H-cyclopenta[d]pyrimidin, und
2-(3,4-Dimethoxyphenyl)-4-cyclohexylamino-6,7-dihydro-5H-cyclopenta[d]pyrimidin, und ihre physiologisch verträglichen Salze.

12. Verbindungen der Formel I gemäß einem oder mehreren der Anspüche 1 bis 11, **dadurch gekennzeichnet gekennzeichnet**, daß sie aus den folgenden Verbindungen ausgewählt sind:

2-(3,5-Dichlorphenyl)-4-(trans-4-hydroxycyclohexylamino)-6,7-dihydro-5H-cyclopenta[d]pyrimidin,
2-(4-Chlorphenyl)-4-(trans-4-hydroxycyclohexylamino)-6,7-dihydro-5H-cyclopenta[d]pyrimidin,
2-(4-Methylphenyl)-4-(trans-4-hydroxycyctohexylamino)-6,7-dihydro-5H-cyclopenta[d]pyrimidin,
und ihre physiologisch verträglichen Salze.

13. Verbindungen der Formel I gemäß einem oder mehreren der Anspüche 1 bis 4, **dadurch gekennzeichnet gekennzeichnet**, daß sie aus den folgenden Verbindungen ausgewählt sind:

2-(4-Chlorphenyl)-4-(2-hydroxyethylamino)-6,7-dihydro-5H-cyclopenta[d]pyrimidin,
2-(4-Chlorphenyl)-4-((3-pyridylmethyl)amino)-6,7-dihydro-5H-cyclopenta[d]pyrimidin,
2-(3-Chlorphenyl)-4-(2-hydroxyethylamino)-6,7-dihydro-5H-cyclopenta[d]pyrimidin,
2-(3-Chlorphenyl)-4-((3-pyridylmethyl)amino)-6,7-dihydro-5H-cyclopenta[d]pyrimidin,
2-(3-Chlorphenyl)-4-(2-(3-methoxyphenyl)-ethylamino)-6,7-dihydro-5H-cyclopenta[d]pyrimidin,
2-(4-Chlorphenyl)-4-(2-(3-methoxyphenyl)-ethylamino)-6,7-dihydro-5H-cyclopenta[d]pyrimidin,
2-(4-Chlorphenyl)-4-(2-methoxyethylamino)-6,7-dihydro-5H-cyclopenta[d]pyrimidin,
2-(4-Chlorphenyl)-4-isobutylamino-6,7-dihydro-5H-cyclopenta[d]pyrimidin,

2-(4-Chlorphenyl)-4-butylamino-6,7-dihydro-5H-cyclopenta[d]pyrimidin,
2-(3-Chlorphenyl)-4-butylamino-6,7-dihydro-5H-cyclopenta[d]pyrimidin,
2-(4-Methylphenyl)-4-(2-hydroxyethylamino)-6,7-dihydro-5H-cyclopenta[d]pyrimidin,
2-(4-Methylphenyl)-4-butylamino-6,7-dihydro-5H-cyclopenta[d]pyrimidin,
2-(4-Methylphenyl)-4-((3-pyridylmethyl)amino)-6,7-dihydro-5H-cyclopenta[d]pyrimidin,
2-(3,5-Dichlorphenyl)-4-(4-hydroxybutylamino)-6,7-dihydro-5H-cyclopenta[d]pyrimidin,
2-(3,5-Dichlorphenyl)-4-butylamino-6,7-dihydro-5H-cyclopenta[d]pyrimidin,
2-(3,5-Dichlorphenyl)-4-((3-pyridylmethyl)amino)-6,7-dihydro-5H-cyclopenta[d]pyrimidin,
2-(4-Methoxyphenyl)-4-(3-hydroxypropylamino)-6,7-dihydro-5H-cyclopenta[d]pyrimidin
2-(4-Methoxyphenyl)-4-butylamino-6,7-dihydro-5H-cyclopenta[d]pyrimidin,
2-(4-Methoxyphenyl)-4-(2-(2-chlorphenyl)-ethylamino)-6,7-dihydro-5H-cyclopenta[d]pyrimidin,
2-(3,4-Dimethoxyphenyl)-4-(3-methoxypropylamino)-6,7-dihydro-5H-cyclopenta[d]pyrimidin,
2-(3,4-Dimethoxyphenyl)-4-butylamino-6,7-dihydro-5H-cyclopenta[d]pyrimidin,
2-(4-Cyanphenyl)-4-(3-methoxypropylamino)-6,7-dihydro-5H-cyclopenta[d]pyrimidin,
2-(4-Cyanphenyl)-4-benzylamino-6,7-dihydro-5H-cyclopenta[d]pyrimidin, und
2-(4-Cyanphenyl)-4-((3-pyridylmethyl)amino)-6,7-dihydro-5H-cyclopenta[d]pyrimidin, und ihre physiologisch verträglichen Salze.

**14.** Verbindungen der Formel I gemäß Anspruch 1 und/oder 2, worin $R^1R^2N$ für einen Rest aus der Reihe Piperidino, Morpholino und Thiomorpholino (und dessen S-Oxid und S,S-Dioxid) steht; in allen ihren stereoisomeren Formen und Mischungen davon in allen Verhältnissen, und ihre physiologisch verträglichen Salze.

**15.** Verbindungen der Formel I gemäß einem oder mehreren der Ansprüche 1 bis 14, worin $R^3$ für substituiertes Phenyl steht; in allen ihren stereoisomeren Formen und Mischungen davon in allen Verhältnissen, und ihre physiologisch verträglichen Salze.

**16.** Verbindungen der Formel I gemäß einem oder mehreren der Ansprüche 1 bis 15, worin $R^3$ für Phenyl steht, das durch einen oder zwei Substituenten aus der Reihe $(C_1-C_4)$-Alkyl und Halogen substituiert ist; in allen ihren stereoisomeren Formen und Mischungen davon in allen Verhältnissen, und ihre physiologisch verträglichen Salze.

**17.** Verfahren zur Herstellung von Verbindungen der Formel I gemäß einem oder mehreren der Ansprüche 1 bis 16, **dadurch gekennzeichnet, daß** ein
4-Hydroxypyrimidin der Formel IV aktiviert wird und dann mit einem Amin der Formel VI umgesetzt wird,

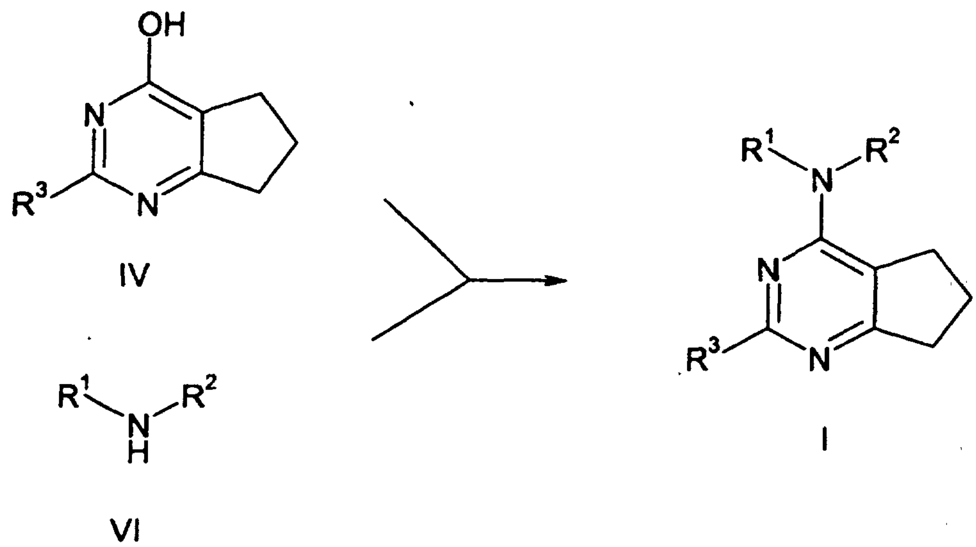

wobei $R^1$, $R^2$ und $R^3$ die in den Ansprüchen 1 bis 16 angegebenen Bedeutungen haben.

**18.** Verbindungen der Formel I gemäß einem oder mehreren der Ansprüche 1 bis 16 und/oder ihre physiologisch

verträglichen Salze zur Verwendung als Arzneimittel.

**19.** Pharmazeutisches Präparat, **dadurch gekennzeichnet, daß** es eine oder mehrere Verbindungen der Formel I gemäß einem oder mehreren der Ansprüche 1 bis 16 und/oder ihre physiologisch verträglichen Salze und einen pharmazeutisch verträglichen Träger enthält.

**20.** Verbindungen der Formel I gemäß einem oder mehreren der Ansprüche 1 bis 16 und/oder ihre physiologisch verträglichen Salze zur Verwendung als Aktivatoren der löslichen Guanylatcyclase.

**21.** Verbindungen der Formel I gemäß einem oder mehreren der Ansprüche 1 bis 16 und/oder ihre physiologisch verträglichen Salze zur Verwendung in der Therapie oder Prophylaxe von Herz-Kreislauf-Erkrankungen, endothelialer Dysfunktion, diastolischer Dysfunktion, Atherosklerose, Bluthochdruck, Angina pectoris, Thrombosen, Restenosen, Myocardinfarkt, Schlaganfällen, Herzinsuffizienz, Pulmonalhypertonie, erektiler Dysfunktion, Asthma bronchiale, chronischer Niereninsuffizienz, Diabetes oder Leberzirrhose oder zur Verbesserung einer eingeschränkten Lemfähigkeit oder Gedächtnisleistung.

## Claims

**1.** A compound of the formula I,

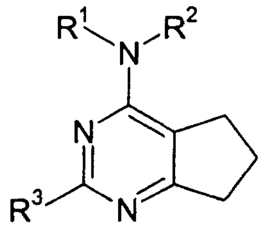

I

in which

$R^1$ and $R^2$, which are independent of one another and can be identical or different, are hydrogen, or $(C_1-C_8)$-alkyl which can be substituted by one or more identical or different substituents from the group consisting of hydroxyl, $(C_1-C_4)$-alkoxy, $(C_1-C_4)$ -alkyl-$S(O)_m$-, phenyl, naphthyl and pyridyl, or $(C_3-C_9)$-cycloalkyl which can be substituted by one or more identical or different substituents from the group consisting of $(C_1-C_4)$-alkyl, hydroxyl, amino and benzyl, or the radical of a 5-membered to 7-membered saturated heterocyclic ring which contains one or two identical or different hetero ring members from the group consisting of O, $NR^{10}$ and $S(O)_m$ and which can be substituted by one or more identical or different substituents from the group consisting of $(C_1-C_4)$-alkyl, hydroxyl and aryl-$(C_1-C_4)$-alkyl-,
where radicals phenyl, naphthyl, pyridyl and benzyl contained in the radicals $R^1$ or $R^2$ can be unsubstituted or can be substituted in the aromatic ring by one or more identical or different substituents from the group consisting of halogen, $(C_1-C_4)$ -alkyl, phenyl, $CF_3$, $NO_2$, OH, -O- $(C_1-C_4)$ -alkyl, -O- $(C_2-C_4)$ -alkyl-O- $(C_1-C_4)$ -alkyl, $(C_1-C_2)$-alkylenedioxy, $NH_2$, -NH-$(C_1-C_4)$-alkyl, -N($(C_1-C_4)$-alkyl)$_2$, -NH-CHO, -NH-CO- $(C_1-C_4)$ -alkyl, -CN, -CO-$NH_2$, -CO-NH-$(C_1-C_4)$-alkyl, -CO-N ($(C_1-C_4)$ -alkyl)$_2$, -CO-OH, -CO-O- $(C_1-C_4)$-alkyl, -CHO and -CO-$(C_1-C_4)$ -alkyl, but where $R^1$ and $R^2$ cannot both simultaneously be hydrogen;
or
the radical $R^1R^2N$ is a radical, bonded via a ring nitrogen atom, of a 5-membered to 7-membered saturated heterocyclic ring which, in addition to the nitrogen atom carrying the radicals $R^1$ and $R^2$, can contain a further hetero ring member from the group consisting of O and $S(O)_m$ and which can be substituted by one or more identical or different substituents from the group consisting of $(C_1-C_4)$ -alkyl, hydroxyl, $(C_1-C_4)$ -alkoxy and $R^{11}R^{12}N$;
$R^3$ is aryl but cannot be unsubstituted phenyl;
$R^{10}$ is hydrogen, $(C_1-C_4)$ -alkyl, aryl- $(C_1-C_4)$ -alkyl-, hydroxy- $(C_1-C_4)$ -alkyl-, hydroxycarbonyl- $(C_1-C_4)$ -alkyl-, ($(C_1-C_4)$ -alkoxycarbonyl) - $(C_1-C_4)$ -alkyl-, $R^{11}R^{12}N$-CO-$(C_1-C_4)$ -alkyl-, $R^{13}$-$SO_2$- or aryl;
$R^{11}$ and $R^{12}$ are identical or different radicals from the group consisting of hydrogen and $(C_1-C_4)$ -alkyl;

$R^{13}$ is $(C_1-C_4)$ -alkyl, aryl or $R^{11}R^{12}N$;
aryl is phenyl, naphthyl or heteroaryl, which can all be substituted by one or more identical or different substituents from the group consisting of halogen, $(C_1-C_4)$-alkyl, phenyl, $CF_3$, $NO_2$, OH, -O- $(C_1-C_4)$ -alkyl, -O- $(C_2-C_4)$ -alkyl-O- $(C_1-C_4)$ -alkyl, $(C_1-C_2)$ -alkylenedioxy, $NH_2$, -NH- $(C_1-C_4)$ -alkyl, -N $((C_1-C_4)$ -alkyl$)_2$, -NH-CHO, -NH-CO- $(C_1-C_4)$ -alkyl, -CN, -CO-$NH_2$, -CO-NH- $(C_1-C_4)$ -alkyl, -CO-N$((C_1-C_4)$-alkyl$)_2$, -CO-OH, -CO-O- $(C_1-C_4)$ -alkyl, -CHO and -CO-$(C_1-C_4)$-alkyl;
heteroaryl is the radical of a monocyclic 5-membered or 6-membered aromatic heterocycle or of a bicyclic 8-membered to 10-membered aromatic heterocycle, each of which contain one or more identical or different ring heteroatoms from the group consisting of N, O and S;
m is 0, 1 or 2;
suitable nitrogen atoms in the compounds of the formula I can also be present as N-oxides;
in all its stereoisomeric forms and mixtures thereof in all ratios, and its physiologically tolerable salts.

**2.** A compound of the formula I as claimed in claim 1, in which
one of the radicals $R^1$ and $R^2$ is $(C_1-C_8)$ -alkyl which can be substituted by one or more identical or different substituents from the group consisting of hydroxyl, $(C_1-C_4)$ -alkoxy, $(C_1-C_4)$ -alkyl-S $(O)_m$-, unsubstituted or substituted phenyl and unsubstituted or substituted naphthyl, or is $(C_3-C_9)$-cycloalkyl which can be substituted by one or more identical or different substituents from the group consisting of $(C_1-C_4)$-alkyl, hydroxyl, amino and unsubstituted or substituted benzyl; and the other of the radicals $R^1$ and $R^2$ is hydrogen, or $(C_1-C_8)$ -alkyl which can be substituted by one or more identical or different substituents from the group consisting of hydroxyl, $(C_1-C_4)$-alkoxy, $(C_1-C_4)$-alkyl-S$(O)_m$-, unsubstituted or substituted phenyl and unsubstituted or substituted naphthyl, or is $(C_3-C_9)$-cycloalkyl which can be substituted by one or more identical or different substituents from the group consisting of $(C_1-C_4)$ -alkyl, hydroxyl, amino and unsubstituted or substituted benzyl;
or
$R^1R^2N$ is a radical, bonded via a ring nitrogen atom, of a 5-membered, 6-membered or 7-membered saturated heterocyclic ring which, in addition to the nitrogen atom carrying the radicals $R^1$ and $R^2$, can additionally contain as a further hetero ring member an oxygen atom or a group $S(O)_m$ and which can be substituted by one or more identical or different substituents from the group consisting of $(C_1-C_4)$ -alkyl, hydroxyl, $(C_1-C_4)$ -alkoxy and $R^{11}R^{12}N$; in all its stereoisomeric forms and mixtures thereof in all ratios, or its physiologically tolerable salts.

**3.** A compound of the formula 1 as claimed in claim 1 and/or 2, in which one of the radicals $R^1$ and $R^2$ is $(C_1-C_4)$-alkyl which can be substituted by one or more identical or different substituents from the group consisting of hydroxyl, $(C_1-C_4)$-alkoxy, $(C_1-C_4)$ - alkyl-S$(O)_m$-, unsubstituted or substituted phenyl and unsubstituted or substituted naphthyl, or $(C_3-C_9)$-cycloalkyl which can be substituted by one or more identical or different substituents from the group consisting of $(C_1-C_4)$-alkyl, hydroxyl, amino and unsubstituted or substituted benzyl, and the other of the radicals $R^1$ and $R^2$ is hydrogen, or the radicals $R^1$ and $R^2$ are identical or different $(C_1-C_4)$ -alkyl which can be substituted by one or more identical or different substituents from the group consisting of hydroxyl, $(C_1-C_4)$ -alkoxy, $(C_1-C_4)$ -alkyl-S $(O)_m$-, unsubstituted or substituted phenyl and unsubstituted or substituted naphthyl;
in all its stereoisomeric forms and mixtures thereof in all ratios, or its physiologically tolerable salts.

**4.** A compound of the formula I as claimed in claim 1 and/or 2, in which one of the radicals $R^1$ and $R^2$ is $(C_1-C_8)$ -alkyl which can be substituted by one or more identical or different substituents from the group consisting of hydroxyl, $(C_1-C_4)$ -alkoxy, $(C_1-C_4)$ -alkyl-S$(O)_m$-, unsubstituted or substituted phenyl and unsubstituted or substituted naphthyl, or $(C_3-C_9)$-cycloalkyl which can be substituted by one or more identical or different substituents from the group consisting of $(C_1-C_4)$-alkyl, hydroxyl, amino and unsubstituted or substituted benzyl; and the other of the radicals $R^1$ and $R^2$ is hydrogen;
in all its stereoisomeric forms and mixtures thereof in all ratios, or its physiologically tolerable salts.

**5.** A compound of the formula I as claimed in one or more of claims 1 to 4, in which one of the radicals $R^1$ and $R^2$ is $(C_3-C_9)$-cycloalkyl which can be substituted by one or more identical or different substituents from the group consisting of $(C_1-C_4)$-alkyl, hydroxyl, amino and benzyl, and the other of the radicals $R^1$ and $R^2$ is hydrogen;
in all its stereoisomeric forms and mixtures thereof in all ratios, or its physiologically tolerable salts.

**6.** A compound of the formula I as claimed in one or more of claims 1 to 5, in which a $(C_3-C_9)$ -cycloalkyl radical represented by $R^1$ or $R^2$ is an unsubstituted or substituted radical from the group consisting of cyclopentyl and cyclohexyl; in all its stereoisomeric forms and mixtures thereof in all ratios, or its physiologically tolerable salts.

**7.** A compound of the formula I as claimed in one or more of claims 1 to 6, in which a substituted cycloalkyl radical

represented by $R^1$ or $R^2$ is substituted by one or more radicals from the group consisting of $(C_1-C_4)$-alkyl, hydroxyl and amino; in all its stereoisomeric forms and mixtures thereof in all ratios, or its physiologically tolerable salts.

**8.** A compound of the formula I as claimed in one or more of claims 1 to 7, in which a substituted cycloalkyl radical is substituted by one or two identical or different substituents; in all its stereoisomeric forms and mixtures thereof in all ratios, or its physiologically tolerable salts.

**9.** A compound of the formula I as claimed in one or more of claims 1 to 8, in which a substituted cycloalkyl radical represented by $R^1$ or $R^2$ is substituted by a hydroxyl group; in all its stereoisomeric forms and mixtures thereof in all ratios, or its physiologically tolerable salts.

**10.** A compound of the formula I as claimed in one or more of claims 1 to 9, in which a substituted cycloalkyl radical is a 4-hydroxycyclohexyl radical; in all its stereoisomeric forms and mixtures thereof in all ratios, or its physiologically tolerable salts.

**11.** A compound of the formula I as claimed in one or more of claims 1 to 10, selected from the following compounds:

2-(4-chlorophenyl)-4-cyclopentylamino-6,7-dihydro-5H-cyclopenta[d]pyrimidine,
2-(3,5-dichlorophenyl)-4-(trans-4-hydroxycyclohexylamino)-6,7-dihydro-5H-cyclopenta[d]pyrimidine,
2-(3-chlorophenyl)-4-cyclopentylamino-6,7-dihydro-5H-cyclopenta[d]pyrimidine,
2-(4-chlorophenyl)-4-(trans-4-hydroxycyclohexylamino)-6,7-dihydro-5H-cyclopenta[d]pyrimidine,
2-(4-methylphenyl)-4-cyclopentylamino-6,7-dihydro-5H-cyclopenta[d]pyrimidine,
2-(4-methylphenyl)-4-cyclohexylamino-6,7-dihydro-5H-cyclopenta[d]pyrimidine,
2-(4-methylphenyl)-4-(trans-4-hydroxycyclohexylamino)-6,7-dihydro-5H-cyclopenta[d]pyrimidine,
2-(3,5-dichlorophenyl)-4-cyclopentylamino-6,7-dihydro-5H-cyclopenta[d]pyrimidine,
2-(4-methoxyphenyl)-4-cyclopentylamino-6,7-dihydro-5H-cyclopenta[d]pyrimidine,
2-(4-methoxyphenyl)-4-cyclohexylamino-6,7-dihydro-5H-cyclopenta[d]pyrimidine,
2-(3,4-dimethoxyphenyl)-4-cyclopentylamino-6,7-dihydro-5H-cyclopenta[d]pyrimidine,
and
2-(3,4-dimethoxyphenyl)-4-cyclohexylamino-6,7-dihydro-5H-cyclopenta[d]pyrimidine,
or its physiologically tolerable salts.

**12.** A compound of the formula I as claimed in one or more of claims 1 to 11, selected from the following compounds:

2-(3,5-dichlorophenyl)-4-(trans-4-hydroxycyclohexylamino)-6,7-dihydro-5H-cyclopenta[d]pyrimidine,
2-(4-chlorophenyl)-4-(trans-4-hydroxycyclohexylamino)-6,7-dihydro-5H-cyclopenta[d]pyrimidine,
2-(4-methylphenyl)-4-(trans-4-hydroxycyclohexylamino)-6,7-dihydro-5H-cyclopenta[d]pyrimidine,

or its physiologically tolerable salts.

**13.** A compound of the formula I as claimed in one or more of claims 1 to 4, selected from the following compounds:

2-(4-chlorophenyl)-4-(2-hydroxyethylamino)-6,7-dihydro-5H-cyclopenta[d]pyrimidine,
2-(4-chlorophenyl)-4-((3-pyridylmethyl)amino)-6,7-dihydro-5H-cyclopenta[d]pyrimidine,
2-(3-chlorophenyl)-4-(2-hydroxyethylamino)-6,7-dihydro-5H-cyclopenta[d]pyrimidine,
2-(3-chlorophenyl)-4-((3-pyridylmethyl)amino)-6,7-dihydro-5H-cyclopenta[d]pyrimidine,
2-(3-chlorophenyl)-4-(2-(3-methoxyphenyl)ethylamino)-6,7-dihydro-5H-cyclopenta[d]pyrimidine,
2-(4-chlorophenyl)-4-(2-(3-methoxyphenyl)ethylamino)-6,7-dihydro-5H-cyclopenta[d]pyrimidine,
2-(4-chlorophenyl)-4-(2-methoxyethylamino)-6,7-dihydro-5H-cyclopenta[d]pyrimidine,
2-(4-chlorophenyl)-4-isobutylamino-6,7-dihydro-5H-cyclopenta[d]pyrimidine,
2-(4-chlorophenyl)-4-butylamino-6,7-dihydro-5H-cyclopenta [d] pyrimidine,
2-(3-chlorophenyl)-4-butylamino-6,7-dihydro-5H-cyclopenta[d]pyrimidine,
2-(4-methylphenyl)-4-(2-hydroxyethylamino)-6,7-dihydro-5H-cyclopenta[d]pyrimidine,
2-(4-methylphenyl)-4-butylamino-6,7-dihydro-5H-cyclopenta [d] pyrimidine,
2-(4-methylphenyl)-4-((3-pyridylmethyl)amino)-6,7-dihydro-5H-cyclopenta[d]pyrimidine,
2-(3,5-dichlorophenyl)-4-(4-hydroxybutylamino)-6,7-dihydro-5H-cyclopenta[d]pyrimidine,
2-(3,5-dichlorophenyl)-4-butylamino-6,7-dihydro-5H-cyclopenta[d]pyrimidine,
2-(3,5-dichlorophenyl)-4-((3-pyridylmethyl)amino)-6,7-dihydro-5H-cyclopenta[d]pyrimidine,

2-(4-methoxyphenyl)-4-(3-hydroxypropylamino)-6,7-dihydro-5H-cyclopenta[d]pyrimidine,
2-(4-methoxyphenyl)-4-butylamino-6,7-dihydro-5H-cyclopenta[d]pyrimidine,
2-(4-methoxyphenyl)-4-(2-(2-chlorophenyl)ethylamino)-6,7-dihydro-5H-cyclopenta[d]pyrimidine,
2-(3,4-dimethoxyphenyl)-4-(3-methoxypropylamino)-6,7-dihydro-5H-cyclopenta[d]pyrimidine,
2-(3,4-dimethoxyphenyl)-4-butylamino-6,7-dihydro-5H-cyclopenta[d]pyrimidine,
2-(4-cyanophenyl)-4-(3-methoxypropylamino)-6,7-dihydro-5H-cyclopenta[d]pyrimidine,
2-(4-cyanophenyl)-4-benzylamino-6,7-dihydro-5H-cyclopenta[d]pyrimidine, and
2-(4-cyanophenyl)-4-((3-pyridylmethyl)amino)-6,7-dihydro-5H-cyclopenta[d]pyrimidine,

or its physiologically tolerable salts.

**14.** A compound of the formula I as claimed in claim 1 and/or 2, in which $R^1R^2N$ is a radical from the group consisting of piperidino, morpholino and thiomorpholino (and its S-oxide and S,S-dioxide); in all its stereoisomeric forms and mixtures thereof in all ratios, or its physiologically tolerable salts.

**15.** A compound of the formula I as claimed in one or more of claims 1 to 14, in which $R^3$ is substituted phenyl; in all its stereoisomeric forms and mixtures thereof in all ratios, or its physiologically tolerable salts.

**16.** A compound of the formula I as claimed in one or more of claims 1 to 15, in which $R^3$ is phenyl which is substituted by one or two substituents from the group consisting of $(C_1\text{-}C_4)$ -alkyl and halogen; in all its stereoisomeric forms and mixtures thereof in all ratios, or its physiologically tolerable salts.

**17.** A process for the preparation of compounds of the formula I as claimed in one or more of claims 1 to 16, which comprises activating a 4-hydroxypyrimidine of the formula IV and then reacting it with an amine of the formula VI,

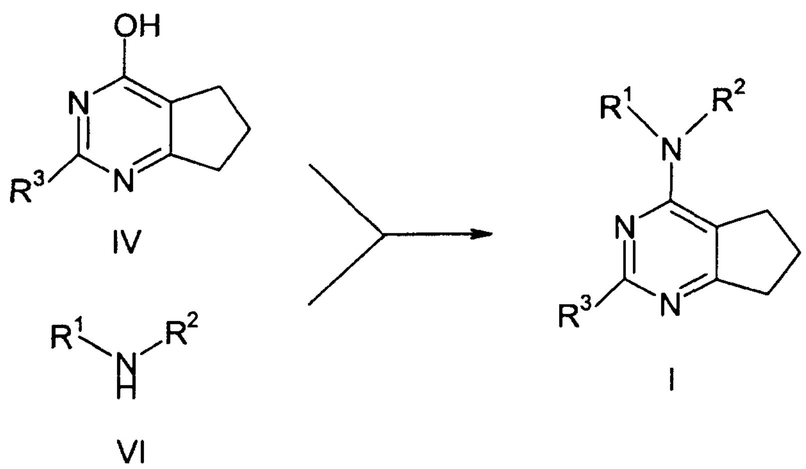

where $R^1$, $R^2$ and $R^3$ have the meanings indicated in claims 1 to 16.

**18.** A compound of the formula I as claimed in one or more of claims 1 to 16 and/or its physiologically tolerable salts for use as a pharmaceutical.

**19.** A pharmaceutical preparation, which contains one or more compounds of the formula I as claimed in one or more of claims 1 to 16 and/or its/their physiologically tolerable salts and a pharmaceutically tolerable carrier.

**20.** A compound of the formula I as claimed in one or more of claims 1 to 16 and/or its physiologically tolerable salts for use as activators of soluble guanylate cyclase.

**21.** A compound of the formula I as claimed in one or more of claims 1 to 16 and/or its physiologically tolerable salts for use in the therapy or prophylaxis of cardiovascular disorders, endothelial dysfunction, diastolic dysfunction, atherosclerosis, high blood pressure, angina pectoris, thromboses, restenoses, myocardial infarct, strokes, cardiac insufficiency, pulmonary hypertension, erectile dysfunction, bronchial asthma, chronic renal insufficiency, diabetes or liver cirrhosis or for improving restricted learning capacity or memory power.

## Revendications

**1.** Composés de formule I,

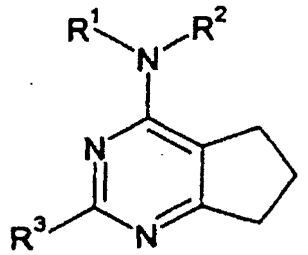

I

dans laquelle

$R^1$ et $R^2$, qui sont indépendants l'un de l'autre et qui peuvent être identiques ou différents, représentent hydrogène ou alkyle en $C_1$ à $C_8$, qui peut être substitué par un ou plusieurs substituants identiques ou différents de la série hydroxy, alcoxy en $C_1$ à $C_4$, (alkyle en $C_1$ à $C_4$) -S $(O)_m$-, phényle, naphtyle et pyridyle, ou représentent cycloalkyle en $C_3$ à $C_9$, qui peut être substitué par un ou plusieurs substituants identiques ou différents de la série alkyle en $C_1$ à $C_4$, hydroxy, amino et benzyle, ou représentent un radical d'un hétérocycle saturé de 5 à 7 chaînons, qui contient un ou deux chaînons d'hétérocycle identiques ou différents de la série O, $NR^{10}$ et $S(O)_m$ et qui peut être substitué par un ou plusieurs substituants identiques ou différents de la série alkyle en $C_1$ à $C_4$, hydroxy et aryl-(alkyle en $C_1$ à $C_4$) -,
où les radicaux phényle, naphtyle, pyridyle et benzyle contenus dans les radicaux $R^1$ ou $R^2$ peuvent être non substitués ou être substitués dans le cycle aromatique par un ou plusieurs substituants identiques ou différents de la série halogène, alkyle en $C_1$ à $C_4$, phényle, $CF_3$, $NO_2$, OH, -O- (alkyle en $C_1$ à $C_4$), -O- (alkyle en $C_2$ à $C_4$)-O-(alkyle en $C_1$ à $C_4$), (alkylène en $C_1$ à $C_2$) dioxy, $NH_2$, -NH-(alkyle en $C_1$ à $C_4$), -N(alkyle en $C_1$ à $C_4)_2$, -NH-CHO, -NH-CO-(alkyle en $C_1$ à $C_4$), -CN, -CO-$NH_2$, -CO-NH-(alkyle en $C_1$ à $C_4$), -CO-N(alkyle en $C_1$ à $C_4)_2$, -CO-OH, -CO-O-(alkyle en $C_1$ à $C_4$), -CHO et -CO- (alkyle en $C_1$ à $C_4$), mais $R^1$ et $R^2$ ne peuvent pas représenter simultanément hydrogène ;
ou
le radical $R^1R^2N$ représente un radical, lié via un atome d'azote de cycle, d'un hétérocycle saturé de 5 à 7 chaînons, qui peut contenir, outre l'atome d'azote portant les radicaux $R^1$ et $R^2$, un autre chaînon d'hétérocycle de la série O et S $(O)_m$ et qui peut être substitué par un ou plusieurs substituants identiques ou différents de la série alkyle en $C_1$ à $C_4$, hydroxy, alcoxy en $C_1$ à $C_4$ et $R^{11}R^{12}N$ ;
$R^3$ représente aryle, mais ne peut pas représenter un phényle non substitué ;
$R^{10}$ représente hydrogène, alkyle en $C_1$ à $C_4$, aryl-(alkyle en $C_1$ à $C_4$), hydroxyalkyle en $C_1$ à $C_4$, hydroxycarbonyl-(alkyle en $C_1$ à $C_4$), ((alcoxy en $C_1$ à $C_4$) carbonyl) (alkyle en $C_1$ à $C_4$) -, $R^{11}R^{12}$N-CO- (alkyle en $C_1$ à $C_4$) -, $R^{13}$-$SO_2$- ou aryle ;
$R^{11}$ et $R^{12}$ représentent des radicaux identiques ou différents de la série hydrogène et alkyle en $C_1$ à $C_4$ ;
$R^{13}$ représente alkyle en $C_1$ à $C_4$, aryle ou $R^{11}R^{12}N$ ;
aryle représente phényle, naphtyle ou hétéroaryle, qui peuvent tous être substitués par un ou plusieurs substituants identiques ou différents de la série halogène, alkyle en $C_1$ à $C_4$, phényle, $CF_3$, $NO_2$, OH, -O-alkyle en $C_1$ à $C_4$, -O- (alkyle en $C_2$ à $C_4$) -O- (alkyle en $C_1$ à $C_4$, (alkylène en $C_1$ à $C_2$)dioxy, $NH_2$, -NH-alkyle en $C_1$ à $C_4$, -N(alkyle en $C_1$ à $C_4)_2$, -NH-CHO, -NH-CO- (alkyle en $C_1$ à $C_4$), -CN, -CO-$NH_2$, -CO-NH-(alkyle en $C_1$ à $C_4$), -CON(alkyle en $C_1$ à $C_4)_2$, -CO-OH, -CO-O-alkyle en $C_1$ à $C_4$, -CHO et -CO- (alkyle en $C_1$ à $C_4$) ;
hétéroaryle représente le radical d'un hétérocycle aromatique, monocyclique de 5 ou 6 chaînons ou d'un hétérocycle aromatique, bicyclique, de 8 à 10 chaînons, qui contient à chaque fois un ou plusieurs hétéroatomes de cycle identiques ou différents de la série N, O et S ;
m représente 0, 1 ou 2 ;
les atomes d'azote appropriés dans les composés de formule I pouvant également se trouver sous forme de N-oxyde ;
dans toutes leurs formes stéréo-isomères et les mélanges de ceux-ci dans tous les rapports, et leurs sels physiologiquement acceptables.

**2.** Composés de formule I selon la revendication 1, dans laquelle

un des radicaux $R^1$ et $R^2$ représente alkyle en $C_1$ à $C_8$, qui peut être substitué par un ou plusieurs substituants identiques ou différents de la série hydroxy, alcoxy en $C_1$ à $C_4$, (alkyle en $C_1$ à $C_4$) - $S(O)_m$-, phényle non substitué ou substitué ou naphtyle non substitué ou substitué, ou représente cycloalkyle en $C_3$ à $C_9$, qui peut être substitué par un ou plusieurs substituants identiques ou différents de la série alkyle en $C_1$ à $C_4$, hydroxy, amino et benzyle non substitué ou substitué, et l'autre radical des radicaux $R^1$ et $R^2$ représente hydrogène ou alkyle en $C_1$ à $C_8$, qui peut être substitué par un ou plusieurs substituants identiques ou différents de la série hydroxy, alcoxy en $C_1$ à $C_4$, (alkyle en $C_1$ à $C_4$) -S $(O)_m$-, phényle non substitué ou substitué et naphtyle non substitué ou substitué, ou représente cycloalkyle en $C_3$ à $C_9$, qui peut être substitué par un ou plusieurs substituants identiques ou différents de la série alkyle en $C_1$ à $C_4$, hydroxy, amino et benzyle non substitué ou substitué ;
ou
$R^1R^2N$ représente un radical, lié via un atome d'azote du cycle, d'un hétérocycle saturé comprenant 5, 6 ou 7 chaînons, qui peut encore contenir, outre l'atome d'azote portant les radicaux $R^1$ et $R^2$, comme autre chaînon d'hétérocycle, un atome d'oxygène ou un groupe $S(O)_m$ et qui peut être substitué par un ou plusieurs substituants identiques ou différents de la série alkyle en $C_1$ à $C_4$, hydroxy, alcoxy en $C_1$ à $C_4$ et $R^{11}R^{12}N$ ; dans toutes leurs formes stéréo-isomères et les mélanges de ceux-ci dans tous les rapports, et leurs sels physiologiquement acceptables.

**3.** Composés de formule I selon la revendication 1 et/ou 2, dans laquelle
un des radicaux $R^1$ et $R^2$ représente alkyle en $C_1$ à $C_4$, qui peut être substitué par un ou plusieurs substituants identiques ou différents de la série hydroxy, alcoxy en $C_1$ à $C_4$, (alkyle en $C_1$ à $C_4$)-$S(O)_m$-, phényle non substitué ou substitué ou naphtyle non substitué ou substitué, ou représente cycloalkyle en $C_3$ à $C_9$, qui peut être substitué par un ou plusieurs substituants identiques ou différents de la série alkyle en $C_1$ à $C_4$, hydroxy, amino et benzyle non substitué ou substitué, et l'autre radical de $R^1$ et $R^2$ représente hydrogène, ou les radicaux $R^1$ et $R^2$ représentent des radicaux alkyle en $C_1$ à $C_4$ identiques ou différents, qui peuvent être substitués par un ou plusieurs substituants identiques ou différents de la série hydroxy, alcoxy en $C_1$ à $C_4$, (alkyle en $C_1$ à $C_4$) - $S(O)_m$-, phényle non substitué ou substitué et naphtyle non substitué ou substitué ;
dans toutes leurs formes stéréo-isomères et les mélanges de ceux-ci dans tous les rapports et leurs sels physiologiquement acceptables.

**4.** Composés de formule I selon la revendication 1 et/ou 2, dans laquelle un des radicaux $R^1$ et $R^2$ représente alkyle en $C_1$ à $C_8$, qui peut être substitué par un ou plusieurs substituants identiques ou différents de la série hydroxy, alcoxy en $C_1$ à $C_4$, (alkyle en $C_1$ à $C_4$)-$S(O)_m$-, phényle non substitué ou substitué et naphtyle non substitué ou substitué, ou représente cycloalkyle en $C_3$ à $C_9$, qui peut être substitué par un ou plusieurs substituants identiques ou différents de la série alkyle en $C_1$ à $C_4$, hydroxy, amino et benzyle non substitué ou substitué, et l'autre radical des radicaux $R^1$ et $R^2$ représente hydrogène ;
dans toutes leurs formes stéréo-isomères et les mélanges de ceux-ci dans tous les rapports et leurs sels physiologiquement acceptables.

**5.** Composés de formule I selon l'une ou plusieurs des revendications 1 à 4, dans laquelle un des radicaux $R^1$ et $R^2$ représente cycloalkyle en $C_3$ à $C_9$, qui peut être substitué par un ou plusieurs substituants identiques ou différents de la série alkyle en $C_1$ à $C_4$, hydroxy, amino et benzyle non substitué ou substitué ; et l'autre des radicaux $R^1$ et $R^2$ représente hydrogène ;
dans toutes leurs formes stéréo-isomères et les mélanges de ceux-ci dans tous les rapports et leurs sels physiologiquement acceptables.

**6.** Composés de formule I selon l'une ou plusieurs des revendications 1 à 5, dans laquelle un radical cycloalkyle en $C_3$ à $C_9$ représentant $R^1$ ou $R^2$ est un radical non substitué ou substitué de la série cyclopentyle et cyclohexyle ; dans toutes leurs formes stéréo-isomères et les mélanges de ceux-ci dans tous les rapports et leurs sels physiologiquement acceptables.

**7.** Composés de formule I selon l'une ou plusieurs des revendications 1 à 6, dans laquelle un radical cycloalkyle substitué représentant $R^1$ ou $R^2$ est substitué par un ou plusieurs radicaux de la série alkyle en $C_1$ à $C_4$, hydroxy et amino ; dans toutes leurs formes stéréo-isomères et les mélanges de ceux-ci dans tous les rapports et leurs sels physiologiquement acceptables.

**8.** Composés de formule I selon l'une ou plusieurs des revendications 1 à 7, dans laquelle un radical cycloalkyle substitué est substitué par un ou deux substituants identiques ou différents ; dans toutes leurs formes stéréo-isomères et les mélanges de ceux-ci dans tous les rapports et leurs sels physiologiquement acceptables.

**9.** Composés de formule I selon l'une ou plusieurs des revendications 1 à 8, dans laquelle un radical cycloalkyle substitué représentant $R^1$ ou $R^2$ est substitué par un groupe hydroxy ; dans toutes leurs formes stéréo-isomères et les mélanges de ceux-ci dans tous les rapports et leurs sels physiologiquement acceptables.

**10.** Composés de formule I selon l'une ou plusieurs des revendications 1 à 9, dans laquelle un radical cycloalkyle substitué est un radical 4-hydroxycyclohexyle ; dans toutes leurs formes stéréo-isomères et les mélanges de ceux-ci dans tous les rapports et leurs sels physiologiquement acceptables.

**11.** Composés de formule I selon l'une ou plusieurs des revendications 1 à 10, **caractérisés en ce qu'**ils sont choisis parmi les composés suivants:

2-(4-chlorophényl)-4-cyclopentylamino-6,7-dihydro-5H-cyclopenta[d]pyrimidine,
2-(3,5-dichlorophényl)-4-(trans-4-hydroxycyclohexylamino)-6,7-dihydro-5H-cyclopenta[d]pyrimidine,
2-(3-chlorophényl)-4-cyclopentylamino-6,7-dihydro-5H-cyclopenta[d]pyrimidine,
2-(4-chlorophényl)-4-(trans-4-hydroxycyclohexylamino)-6,7-dihydro-5H-cyclopenta[d]pyrimidine,
2-(4-méthylphényl)-4-cyclopentylamino-6,7-dihydro-5H-cyclopenta[d]pyrimidine,
2-(4-méthylphényl)-4-cyclohexylamino-6,7-dihydro-5H-cyclopenta[d]pyrimidine,
2-(4-méthylphényl)-4-(trans-4-hydroxycyclohexylamino)-6,7-dihydro-5H-cyclopenta[d]pyrimidine,
2-(3,5-dichlorophényl)-4-cyclopentylamino-6,7-dihydro-5H-cyclopenta[d]pyrimidine,
2-(4-méthoxyphényl)-4-cyclopentylamino-6,7-dihydro-5H-cyclopenta[d]pyrimidine,
2-(4-méthoxyphényl)-4-cyclohexylamino-6,7-dihydro-5H-cyclopenta[d]pyrimidine,
2-(3,4-diméthoxyphényl)-4-cyclopentylamino-6,7-dihydro-5H-cyclopenta[d]pyrimidine, et
2-(3,4-diméthoxyphényl)-4-cyclohexylamino-6,7-dihydro-5H-cyclopenta[d]pyrimidine,

et leurs sels physiologiquement acceptables.

**12.** Composés de formule I selon l'une ou plusieurs des revendications 1 à 11, **caractérisés en ce qu'**ils sont choisis parmi les composés suivants :

2-(3,5-dichlorophényl)-4-(trans-4-hydroxycyclohexylamino)-6,7-dihydro-5H-cyclopenta[d]pyrimidine,
2-(4-chlorophényl)-4-(trans-4-hydroxycyclohexylamino)-6,7-dihydro-5H-cyclopenta[d]pyrimidine,
2-(4-méthylphényl)-4-(trans-4-hydroxycyclohexylamino)-6,7-dihydro-5H-cyclopenta[d]pyrimidine,

et leurs sels physiologiquement acceptables.

**13.** Composés de formule I selon l'une ou plusieurs des revendications 1 à 4, **caractérisés en ce qu'**ils sont choisis parmi les composés suivants :

2-(4-chlorophényl)-4-(2-hydroxyéthylamino)-6,7-dihydro-5H-cyclopenta[d]pyrimidine,
2-(4-chlorophényl)-4-((3-pyridylméthyl)amino)-6,7-dihydro-5H-cyclopenta[d]pyrimidine,
2-(3-chlorophényl)-4-(2-hydroxyéthylamino)-6,7-dihydro-5H-cyclopenta[d]pyrimidine,
2-(3-chlorophényl)-4-((3-pyridylméthyl)amino)-6,7-dihydro-5H-cyclopenta[d]pyrimidine,
2-(3-chlorophényl)-4-(2-(3-méthoxyphényl)éthylamino)-6,7-dihydro-5H-cyclopenta[d]pyrimidine,
2-(4-chlorophényl)-4-(2-(3-méthoxyphényl)éthylamino)-6,7-dihydro-5H-cyclopenta[d]pyrimidine,
2-(4-chlorophényl)-4-(2-méthoxyéthylamino)-6,7-dihydro-5H-cyclopenta[d]pyrimidine,
2-(4-chlorophényl)-4-isobutylamino-6,7-dihydro-5H-cyclopenta[d]pyrimidine,
2-(4-chlorophényl)-4-butylamino-6,7-dihydro-5H-cyclopenta[d]pyrimidine,
2-(3-chlorophényl)-4-butylamino-6,7-dihydro-5H-cyclopenta[d]pyrimidine,
2-(4-méthylphényl)-4-(2-hydroxyéthylamino)-6,7-dihydro-5H-cyclopenta[d]pyrimidine,
2-(4-méthylphényl)-4-butylamino-6,7-dihydro-5H-cyclopenta [d] pyrimidine,
2-(4-méthylphényl)-4-((3-pyridylméthyl)amino)-6,7-dihydro-5H-cyclopenta[d]pyrimidine,
2-(3,5-dichlorophényl)-4-(4-hydroxybutylamino)-6,7-dihydro-5H-cyclopenta[d]pyrimidine,
2-(3,5-dichlorophényl)-4-butylamino-6,7-dihydro-5H-cyclopenta [d] pyrimidine,
2-(3,5-dichlorophényl)-4-((3-pyridylméthyl)amino)-6,7-dihydro-5H-cyclopenta[d]pyrimidine,
2-(4-méthoxyphényl)-4-(3-hydroxypropylamino)-6,7-dihydro-5H-cyclopenta[d]pyrimidine
2-(4-méthoxyphényl)-4-butylamino-6,7-dihydro-5H-cyclopenta[d]pyrimidine,
2-(4-méthoxyphényl)-4-(2-(2-chlorophényl)éthylamino)-6,7-dihydro-5H-cyclopenta[d]pyrimidine,
2-(3,4-diméthoxyphényl)-4-(3-méthoxypropylamino)-6,7-dihydro-5H-cyclopenta[d]pyrimidine,

2-(3,4-diméthoxyphényl)-4-butylamino-6,7-dihydro-5H-cyclopenta[d]pyrimidine,
2-(4-cyanophényl)-4-(3-méthoxypropylamino)-6,7-dihydro-5H-cyclopenta[d]pyrimidine,
2-(4-cyanophényl)-4-benzylamino-6,7-dihydro-5H-cyclopenta [d] pyrimidine, et
2-(4-cyanophényl)-4-((3-pyridylméthyl)amino)-6,7-dihydro-5H-cyclopenta[d]pyrimidine, et leurs sels physiologiquement acceptables.

**14.** Composés de formule I selon la revendication 1 et/ou 2, dans laquelle $R^1R^2N$ représente un radical de la série pipéridino, morpholino et thiomorpholino (et leur S-oxyde et S,S-dioxyde) ; dans toutes leurs formes stéréo-isomères et les mélanges de ceux-ci dans tous les rapports et leurs sels physiologiquement acceptables .

**15.** Composés de formule I selon l'une ou plusieurs des revendications 1 à 14, dans laquelle $R^3$ représente phényle substitué ; dans toutes leurs formes stéréo-isomères et les mélanges de ceux-ci dans tous les rapports et leurs sels physiologiquement acceptables.

**16.** Composés de formule I selon l'une ou plusieurs des revendications 1 à 15, dans laquelle $R^3$ représente phényle qui est substitué par un ou deux substituants de la série alkyle en $C_1$ à $C_4$ et halogène ; dans toutes leurs formes stéréo-isomères et les mélanges de ceux-ci dans tous les rapports et leurs sels physiologiquement acceptables.

**17.** Procédé pour la préparation de composés de formule I selon l'une ou plusieurs des revendications 1 à 16, **caractérisé en ce qu'**on active une 4-hydroxypyrimidine de formule IV puis on transforme avec une amine de formule VI,

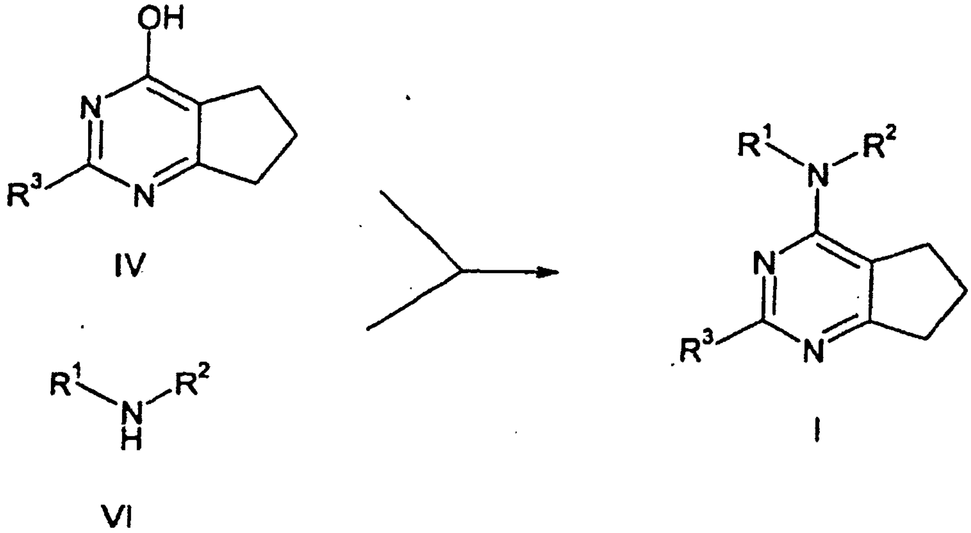

IV

VI

I

$R^1$, $R^2$ et $R^3$ ayant les significations indiquées dans les revendications 1 à 16.

**18.** Composés de formule I selon l'une ou plusieurs des revendications 1 à 16 et/ou leurs sels physiologiquement acceptables destinés à une utilisation comme médicament..

**19.** Préparation pharmaceutique, **caractérisée en ce qu'**elle contient un ou plusieurs composés de formule I selon l'une ou plusieurs des revendications 1 à 16 et/ou leurs sels physiologiquement acceptables et un support physiologiquement acceptable.

**20.** Composés de formule I selon l'une ou plusieurs des revendications 1 à 16 et/ou leurs sels physiologiquement acceptables destinés à une utilisation comme activateurs de la guanylate-cyclase soluble.

**21.** Composés de formule I selon l'une ou plusieurs des revendications 1 à 16 et/ou leurs sels physiologiquement acceptables destinés à une utilisation dans la thérapie ou la prophylaxie de maladies cardio-vasculaires, du dysfonctionnement endothélial, du dysfonctionnement diastolique, de l'athérosclérose, de l'hypertension, de l'angine de poitrine, de thromboses, de resténoses, de l'infarctus du myocarde, d'attaques, d'insuffisance cardiaque, de l'hypertonie pulmonaire, du dysfonctionnement érectile, de l'asthme bronchique, de l'insuffisance rénale chronique, du diabète ou de la cirrhose du foie ou à l'amélioration d'une aptitude limitée à l'apprentissage ou de la mémoire.